# EUROPEAN PATENT SPECIFICATION

(11) **EP 1 718 617 B1**
(45) Date of publication and mention of the grant of the patent: **19.08.2009**
(21) Application number: 05717730.5
(22) Date of filing: 16.02.2005
(51) Int. Cl.: C07D 231/00

(54) **3-SUBSTITUTED 1,5-DIPHENYLPYRAZOLE DERIVATIVES USEFUL AS CB1 MODULATORS**
3-SUBSTITUIERTE 1,5-DIPHENYLPYRAZOLDERIVATE, DIE SICH ALS CB1-MODULATOREN EIGNEN
DÉRIVÉS 1,5-DIPHÉNYLPYRAZOLE-3-SUBSTITUÉ ET LEUR UTILISATION COMME MODULATEURS CB1

(30) Priority: 20.02.2004 GB 0403779; 18.09.2004 GB 0420780
(43) Date of publication of application: 08.11.2006
(73) Proprietor: AstraZeneca AB, 151 85 Södertälje (SE)
(72) Inventor: CHENG, Leifeng, S-SE-431 83 Molndal (SE); LINDSTEDT-ALSTERMARK, Eva-Lotte, S-SE-431 83 Molndal (SE); BOIJE, Anna, Maria, Persdotter, S-SE-431 83 Molndal (SE)
(86) International application number: PCT/GB2005/000534
(87) International publication number: WO 2005/080343

(56) References cited:
- EP-A- 0 658 546
- WO-A-03/020217
- WO-A-20/04050632
- REETI KATOCH-TOUSE ET AL.: "Synthesis, structure-activity relationship, and evaluation of SR141716 analogues:" JOURNAL OF MEDICINAL CHEMISTRY., vol. 46, no. 4, 2003, pages 642-645, XP002335279 USAMERICAN CHEMICAL SOCIETY. WASHINGTON. cited in the application

## Description

### Field of invention

The present invention relates to certain compounds of formula IA, to processes for preparing such compounds, to their use in the treatment of obesity, psychiatric and neurological disorders, to methods for their therapeutic use and to pharmaceutical compositions containing them.

### Background of the invention

It is known that certain CB₁ modulators (known as antagonists or inverse agonists) are useful in the treatment of obesity, psychiatric and neurological disorders (WO01/70700 EP 658,546 and EP 656,354). However, there is a need for CB₁ modulators with improved physicochemical properties and/or DMPK properties and/or pharmacodynamic properties.

Pyrazoles having anti-inflammatory activity are disclosed in WO 95/15316, WO96/38418, WO97/11704, WO99/64415, EP 418 845 and WO2004050632. WO2004050632 discloses 1,1-dimethylethyl [2-[4-[3-[(ethylmethylamino)carbonyl]-1-(4-methoxyphenyl)-1H-pyrazol-5-yl]phenoxy]ethyl]carbamate, 5-[4-(2-aminoethoxy)phenyl]-*N*-ethyl-1-(4-methoxyphenyl)-*N*-methyl-1H-pyrazole-3-carboxamide, 1-[[5-[4-(2-aminoethoxy)phenyl]-1-(4-methoxyphenyl)-1*H*-pyrazol-3-yl]carbonyl]piperidine and 1,1-dimethylethyl [2-[4-[1-(4-methoxyphenyl)-3-(1-piperidinylcarbonyl)-1*H*-pyrazol-5-yl]phenoxy]ethyl]-carbamate. All compounds exemplified in WO2004050632 and salts thereof are excluded from the scope of the compound claims of the present invention. 1,5-Diarylpyrazole-3-carboxamide derivatives are disclosed as having CB₁ modulatory activity in US 5,624,941, WO01/29007, WO2004/052864, WO03/020217, US 2004/0119972, Journal of Medicinal Chemistry, 46(4), 642-645 2003, Bioorganic & Medicinal Chemistry Letters, 14(10), 2393-2396 2004, Biochemical Pharmacology, 60(9), 1315-1323 2000, Journal of Medicinal Chemistry, 42(4), 769-776 1999 and U.S. Pat. Appl. Publ. US 2003199536.

### Description of the invention

The invention relates to a compound of Formula IA 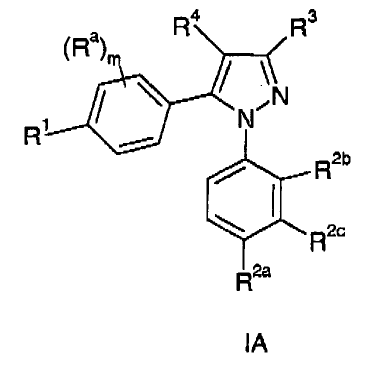 or a pharmaceutically acceptable salt thereof, in which
in which R¹ is a group R⁵S(O)₂O in which R⁵ represents a C₁₋₆alkyl group optionally substituted by one or more fluoro;
R^{a} represents halo and m is 0, 1 or 2;
R^{2a} represents chloro;
R^{2b} represents chloro;
R^{2c} represents H or fluoro;
R³ represents a group CONHNR⁷R⁸ in which NR⁷R⁸ represents piperidino or morpholino or R³ represents a group CONHR⁸ in which R⁸ represents a C₅₋₇cycloalkyl group optionally substituted by a C₁₋₆alkoxycarbonyl group or R³ represents 5-(trifluoromethyl)pyridin-2-yl]amino}carbonyl);
and
R⁴ represents H, a C₁₋₃alkyl group or halo.

Suitable groups in which R¹ represents a group R⁵S(O)₂O in which R⁵ represents a C₁₋₆alkyl group optionally substituted by one or more fluoro include methanesulfonyloxy, ethanesulfonyloxy, n-propylsulfonyloxy, n-butylsulfonyloxy, 3-methylbutane-1-sulfonyloxy, 3,3-dimethylbutane-1-sulfonyloxy, fluoromethylsulfonyloxy, difluoromethylsulfonyloxy, trifluoromethylsulfonyloxy, mono, di or tri (fluoroethyl)sulfonloxy, 3,3,3-trifluoropropyl-1-sulfonyloxy, or 4,4,4-trifluorobutyl-1-sulfonyloxy,

A further particular group of compounds of formula I is represented by formula IB 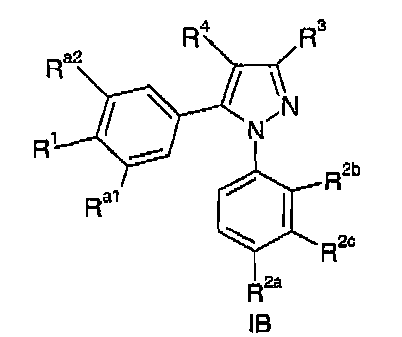 in which R¹ represents a group R⁵S(O)₂O in which R⁵ represents a C₁₋₆alkyl group optionally substituted by one or more fluoro;
R^{a1} represents halo or H; R^{a2} represents halo or H;
R^{2a} represents chloro; R^{2b} represents chloro, R^{2c} represents halo or H;
R³ represents a group CONHNR⁷R⁸ in which NR⁷R⁸ represents piperidino; and
R⁴ represents a C₁₋₃alkyl group.

A particular group of compounds of formula I is represented by formula IC 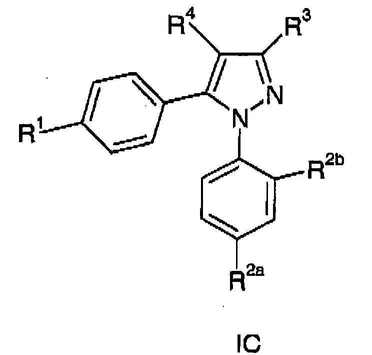 in which R¹ is represents a group R⁵S(O)₂O in which R⁵ represents a C₁₋₆alkyl group optionally substituted by one or more fluoro;
R^{2a} represents chloro;
R^{2b} represents chloro;
R³ represents a group CONHNR⁷R⁸ in which NR⁷R⁸ represents piperidino or morpholino; and
R⁴ represents a C₁₋₃alkyl group or halo.

In a particular group of compounds of formula IC, R¹ is a group R⁵S(O)₂O in which R⁵ represents a C₃₋₆alkyl group substituted by one or more fluoro.

Further values of R¹, R², R³, R⁴ and R^{a} in compounds of formula I, formula IA, formula IB, or formula IC now follow. It will be understood that such values may be used where appropriate with any of the definitions, claims or embodiments defined hereinbefore or hereinafter.
R¹ represents n-butylsulfonyloxy, n-propylsulfonyloxy, 4,4,4-trifluorobutyl-1-sulfonyloxy, 3,3,3-triftuoropropyl-1-sulfonyloxy, 3-methylbutane-1-sulfonyloxy or 3,3-dimethylbutane-1-sulfonyloxy.
R¹ represents n-butylsulfonyloxy, n-propylsulfonyloxy, 4,4,4-trifluorobutyl-1-sulfonyloxy or 3,3,3-trifluoropropyl-1-sulfonyloxy,
R¹ represents n-butylsulfonyloxy, n-propylsulfonyloxy, 4,4,4-trifluorobutyl-1-sulfonyloxy or 3,3,3-trifluoropropyl-1-sulfonyloxy.
R³ represents *N*-(piperidin-1-yl)carbamoyl, 5-(trifluoromethyl)pyridin-2-yl]amino}carbonyl, morpholin-4-ylcarbamoyl or *N*-(1-methoxycarbonyl-1-cyclopentyl)carbamoyl.
R⁴ represents C₁₋₄alkyl or halo. R⁴ represents methyl or bromo,
m is 0 or when m is 1 or 2 R³ represents fluoro,

In a further particular group of compounds of formula I, formula IA, formula IB, or formula IC or any embodiments thereof, R³ represents N-(piperidin-1-yl)carbamoyl. "Pharmaceutically acceptable salt", where such salts are possible, includes both pharmaceutically acceptable acid and base addition salts. A suitable pharmaceutically acceptable salt of a compound of Formula I is, for example, an acid-addition salt of a compound of Formula I which is sufficiently basic, for example an acid-addition salt with an inorganic or organic acid such as hydrochloric, hydrobromic, sulphuric, trifluoroacetic, citric or maleic acid; or, for example a salt of a compound of Formula I which is sufficiently acidic, for example an alkali or alkaline earth metal salt such as a sodium, calcium or magnesium salt, or an ammonium salt, or a salt with an organic base such as methylamine, dimethylamine, trimethylamine, piperidine, morpholine or tris-(2-hydroxyethyl)amine.

Throughout the specification and the appended claims, a given chemical formula or name shall encompass all stereo and optical isomers and racemates thereof as well as mixtures in different proportions of the separated enantiomers, where such isomers and enantiomers exist, as well as pharmaceutically acceptable salts thereof and solvates thereof such as for instance hydrates. Isomers may be separated using conventional techniques, e.g. chromatography or fractional crystalisation. The enantiomers may be isolated by separation of racemate for example by fractional crystallisation, resolution or HPLC. The diastereomers may be isolated by separation of isomer mixtures for instance by fractional crystallisation, HPLC or flash chromatography. Alternatively the stereoisomers may be made by chiral synthesis from chiral starting materials under conditions which will not cause racemisation or epimerisation, or by derivatisation, with a chiral reagent. All stereoisomers are included within the scope of the invention. All tautomers, where possible, are included within the scope of the invention. The present invention also encompasses compounds containing one or more isotopes for example ¹⁴C, ¹¹C or ¹⁹F and their use as isotopically labelled compounds for pharmacological and metabolic studies.

The present invention also describes prodrugs of a compound of formula I that is compounds which are converted into a compound of formula I in vivo.

The following definitions shall apply throughout the specification and the appended claims.

Unless otherwise stated or indicated, the term "alkyl" denotes either a straight or branched alkyl group. Examples of said alkyl include methyl, ethyl, n-propyl, isopropyl, n-butyl, iso-butyl, sec-butyl , t-butyl, pentyl, isopentyl, neopentyl, *tert*-pentyl, hexyl and isohexyl. Preferred alkyl groups are methyl, ethyl, propyl, isopropyl, butyl and tertiary butyl.

Unless otherwise stated or indicated, the term "alkoxy" denotes a group O-alkyl, wherein alkyl is as defined above.

Unless otherwise stated or indicated, the term "halogen" shall mean fluorine, chlorine, bromine or iodine.

Specific compounds of the invention are one or more of the following:
butane-1-sulfonic acid 4-[2-(2,4-dichlorophenyl)-4-methyl-5-(piperidin-1-ylcarbamoyl)-2*H*-pyrazol-3-yl]phenyl ester;
propane-1-sulfonic acid 4-[2-(2,4-dichloro-phenyl)-4-methyl-5-(piperidin-1-ylcarbamoyl)2*H*-pyrazol-3-yl]-phenyl ester;
propane-1-sulfonic acid 4-[2-(2,4-dichlorophenyl)-4-methyl-5-(morpholin-4-ylcarbamoyl)-2*H*-pyrazol-3-yl]phenyl ester;
3,3,3-trifluoropropane-1-sulfonic acid 4-[2-(2,4-dichlorophenyl)-4-methyl-5-(piperidin-1-ylcarbamoyl)-2*H*-pyrazol-3-yl]phenyl ester;
4,4,4-trifluorobutane-1-sulfonic acid 4-[2-(2,4-dichlorophenyl)-4-methyl-5-(piperidin-1-ylcarbamoyl)-2*H*-pyrazol-3-yl]phenyl ester;
propane-1-sulfonic acid-4-[2-(2,4-dichlorophenyl)-4-methyl-5-(pipendin-1-ylcarbamoyl)-2*H*-pyrazol-3-yl]-2,6-difluorophenyl ester;
propane-1-sulfonic acid 4-[2-(2,4-dichlorophenyl)-5-(piperidin-1-ylcarbamoyl)-2*H-*pyrazol-3-yl]phenyl ester;
propane-1-sulfonic acid 4-[4-bromo-2-(2,4-dichlorophenyl)-5-(piperidin-1-ylcarbamoyl)-2*H*-pyrazol-3-yl]phenyl ester;
methyl 1-{[(1-(2,4-dichlorophenyl)-4-methyl-5-{4[(propylsulfonyl)oxy]phenyl}-1*H-*pyrazol-3-yl)carbonyl]amino}cyclopentanecarboxylate;
4-{1-(2,4-dichlorophenyl)-4-methyl-3-[(piperidin-1-ylamino)carbonyl]-1*H*-pyrazol-5-yl}phenyl3-methylbutane-1-sulfonate;
4-{1-(2,4-dichlorophenyl)-4-methyl-3-[(piperidin-1-ylamino)carbonyl]-1*H*-pyrazol-5-yl}phenyl3,3-dimethylbutane-1-sulfonate;
4-[1-(2,4-dichlorophenyl)-4-methyl-3-({[5-(trifluoromethyl)pyridin-2-yl]amino}carbonyl)-1*H*-pyrazol-5-yl]phenyl 3,3,3-trifluoropropane-1-sulfonate;
propane-1-sulfonic acid 4-[2-(2,4-dichloro-3-fluorophenyl)-4-methyl-5-(piperidin-1-ylcarbamoyl)-2*H*-pyrazol-3-yl]phenyl ester; and
or a pharmaceutically acceptable salt thereof.

### Methods of preparation

Compounds of formula IA in which R^{a}, R², R³, R⁴, and m are as previously defined and R¹ represents a group R⁵S(O)₂O may be prepared by reacting a compound of formula IIIA 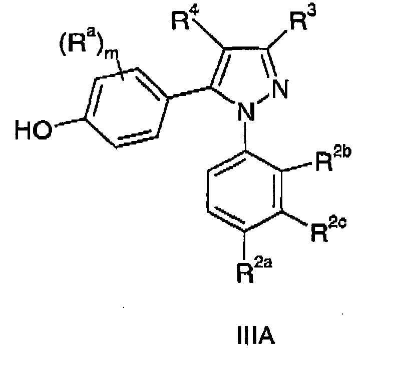 in which R^{a}, R^{2a}, R^{2b}, R^{2c}, R², R³, R⁴ and m are as previously defined with either
a) an alkylating agent of formula R⁹X in which R⁹ represents a C₁₋₃alkyl group substituted by one or more fluoro or C₄₋₆alkyl group optionally substituted by one or more fluoro and X represents a leaving group, for example chloro, bromo or iodo, in an inert solvent, for example acetone, in the presence of a base, for example potassium carbonate, at a temperature in the range of -25°C to 150°C; or
b) an alkylating agent of formula R⁹X in which R⁹ represents a group of formula phenyl(CH₂)ₚ- in which p is 1, 2 or 3 and the phenyl ring is optionally substituted by 1, 2 or 3 groups represented by Z, and X represents a leaving group, for example chloro, bromo or iodo, in an inert solvent, for example acetone, in the presence of a base, for example potassium carbonate, at a temperature in the range of -25°C to 150°C; or
c) a sulphonating agent of formula R⁵SO₂L in which R⁵ is as previously defined and L represents a leaving group, for example chloro, in an inert solvent, for example dichloromethane, in the presence of a base, for example triethylamine, at a temperature in the range of -25°C to 150°C; respectively.

Compounds of formula I in which R^{a}, R¹, R², R⁴, m and n are as previously defined and R³ represents a group X-Y-NR⁷R⁸ in which X is CO , Y is absent or represents NH optionally substituted by a C₁₋₃alkyl group and R⁷ and R⁸ are as previously defined may also be prepared by reacting a compound of formula IV 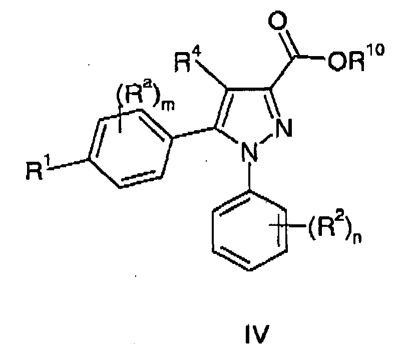 in which R^{a}, R¹, R², R⁴, m and n are as previously defined and R¹⁰ represents a C₁₋₆alkyl group with a compound of formula V

R⁷ R⁸ YNH₂ V

in which Y, R⁷ and R⁸ are as previously defined or a salt thereof in an inert solvent, for example toluene, in the presence of a Lewis acid, for example trimethylaluminium, at a temperature in the range of -25°C to 150°C;

Compounds of formula III, IV and VI may be prepared by methods analogous to the following method. 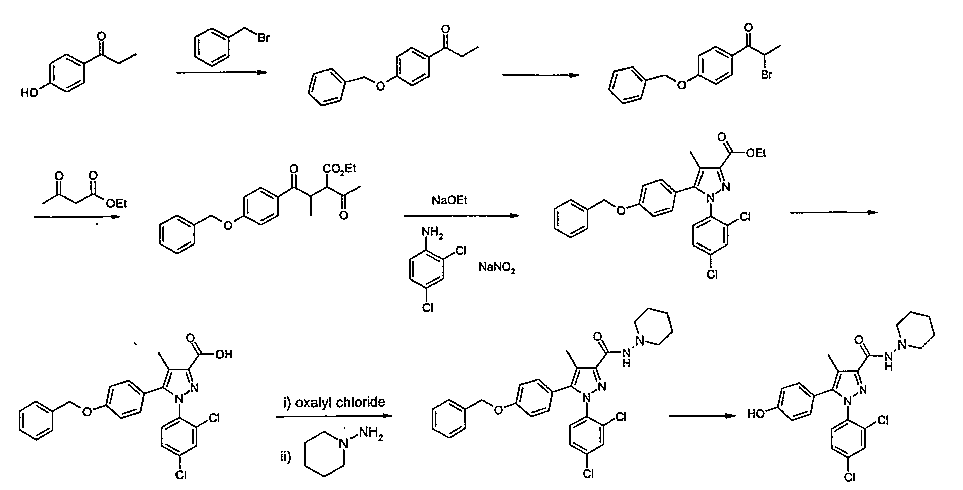 Certain intermediate compounds are believed to be novel and form part of the present invention. It will be appreciated by those skilled in the art that during the reaction sequence certain functional groups will require protection followed by deprotection at an appropriate stage see "Protective Groups in Organic Synthesis", 3rd Edition (1999) by Greene and Wuts.

### Pharmaceutical preparations

The compounds of the invention will normally be administered via the oral, parenteral, intravenous, intramuscular, subcutaneous or in other injectable ways, buccal, recital, vaginal, transdermal and/or nasal route and/or via inhalation, in the form of pharmaceutical preparations comprising the active ingredient or a pharmaceutically acceptable addition salt, in a pharmaceutically acceptable dosage form. Depending upon the disorder and patient to be treated and the route of administration, the compositions may be administered at varying doses.

Suitable daily doses of the compounds of the invention in the therapeutic treatment of humans are about 0.001-10 mg/kg body weight, preferably 0.01-1 mg/kg body weight.

Oral formulations are preferred particularly tablets or capsules which may be formulated by methods known to those skilled in the art to provide doses of the active compound in the range of 0.5mg to 500mg for example 1 mg, 3 mg, 5 mg, 10 mg, 25mg, 50mg, 100mg and 250mg.

According to a further aspect of the invention there is also provided a pharmaceutical formulation including any of the compounds of the invention, or pharmaceutically acceptable derivatives thereof, in admixture with pharmaceutically acceptable adjuvants, diluents and/or carriers.

### Pharmacological properties

The compounds of formula (I) are useful for the treatment of obesity or being overweight, (e.g., promotion of weight loss and maintenance of weight loss), prevention of weight gain (e.g., medication-induced or subsequent to cessation of smoking), for modulation of appetite and/or satiety, eating disorders (e.g. binge eating, anorexia, bulimia and compulsive), cravings (for drugs, tobacco, alcohol, any appetizing macronutrients or non-essential food items), for the treatment of psychiatric disorders such as psychotic and/or mood disorders, schizophrenia and schizo-affective disorder, bipolar disorders, anxiety, anxio-depressive disorders, depression, mania, obsessive-compulsive disorders, impulse control disorders (e.g., Gilles de la Tourette's syndrome), attention disorders like ADD/ADHD, stress, and neurological disorders such as dementia and cognitive and/or memory dysfunction (e.g., amnesia, Alzheimer's disease, Pick's dementia, dementia of ageing, vascular dementia, mild cognitive impairment, age-related cognitive decline, and mild dementia of ageing), neurological and/or neurodegenerative disorders (e.g. Multiple Sclerosis, Raynaud's syndrome, Parkinson's disease, Huntington's chorea and Alzheimer's disease), demyelinisation-related disorders, neuroinflammatory disorders (e.g., Guillain-Barré syndrome).

The compounds are also potentially useful for the prevention or treatment of dependence and addictive disorders and behaviours (e.g., alcohol and/or drug abuse, pathological gambling, kleptomania), drug withdrawal disorders (e.g., alcohol withdrawal with or without perceptual disturbances; alcohol withdrawal delirium; amphetamine withdrawal; cocaine withdrawal; nicotine withdrawal; opioid withdrawal; sedative, hypnotic or anxiolytic withdrawal with or without perceptual disturbances; sedative, hypnotic or anxiolytic withdrawal delirium; and withdrawal symptoms due to other substances), alcohol and/or drug-induced mood, anxiety and/or sleep disorder with onset during withdrawal, and alcohol and/or drug relapse.

The compounds are also potentially useful for the prevention or treatment of neurological dysfunctions such as dystonias, dyskinesias, akathisia, tremor and spasticity, treatment of spinal cord injury, neuropathy, migraine, vigilance disorders, sleep disorders (e.g., disturbed sleep architecture, sleep apnea, obstructive sleep apnea, sleep apnea syndrome), pain disorders, cranial trauma.

The compounds are also potentially useful for the treatment of immune, cardiovascular disorders (e.g. atherosclerosis, arteriosclerosis, angina pectoris, abnormal heart rythms, and arrythmias, congestive heart failure, coronary artery disease, heart disease, hypertension, prevention and treatment of left ventricular hypertrophy, myocardial infarction, transient ischaemic attack, peripheral vascular disease, systemic inflammation of the vasculature, septic chock, stroke, cerebral apoplexy, cerebral infarction, cerebral ischaemia, cerebral thrombosis, cerebral embolism, cerebral hemorrhagia, metabolic disorders (e.g. conditions showing reduced metabolic activity or a decrease in resting energy expenditure as a percentage of total fat-free mass, diabetes mellitus, dyslipidemia, fatty liver, gout, hypercholesterolemia, hyperlipidemia, hypertriglyceridemia, hyperuricacidemia, impaired glucose tolerance, impaired fasting glucose, insulin resistance, insulin resistance syndrome, metabolic syndrome, syndrome X, obesity-hypoventilation syndrome (Pickwickian syndrome), type I diabetes, type II diabetes, low HDL- and/or high LDL-cholesterol levels, low adiponectin levels), reproductive and endocrine disorders (e.g. treatment of hypogonadism in males, treatment of infertility or as contraceptive, menstrual abnormalities/emmeniopathy, polycystic ovarian disease, sexual and reproductive dysfunction in women and men (erectile dysfunction), GH-deficient subjects, hirsutism in females, normal variant short stature) and diseases related to the respiratory (e.g. asthma and chronic obstructive pulmonary disease) and gastrointestinal systems (e.g. dysfunction of gastrointestinal motility or intestinal propulsion, diarrhea, emesis, nausea, gallbladder disease, cholelithiasis, obesity-related gastro-esophageal reflux, ulcers).

The compounds are also potentially useful as agents in treatment of dermatological disorders, cancers (e.g. colon, rectum, prostate, breast, ovary, endometrium, cervix, gallbladder, bile duct), craniopharyngioma, Prader-Willi syndrome, Turner syndrome, Frohlich's syndrome, glaucoma, infectious diseases, urinary tract disorders and inflammatory disorders (e.g. arthritis deformans, inflammation, inflammatory sequelae of viral encephalitis, osteoarthritis) and orthopedic disorders. The compounds are also potentially useful as agents in treatment of (esophageal) achalasia.

In another aspect the present invention provides a compound of formula I as previously defined for use as a medicament.

In a further aspect the present invention provides the use of a compound of formula I in the preparation of a medicament for the treatment or prophylaxis of obesity or being overweight, (e.g., promotion of weight loss and maintenance of weight loss), prevention of weight gain (e.g., medication-induced or subsequent to cessation of smoking), for modulation of appetite and/or satiety, eating disorders (e.g. binge eating, anorexia, bulimia and compulsive), cravings (for drugs, tobacco, alcohol, any appetizing macronutrients or non-essential food items), for the treatment of psychiatric disorders such as psychotic and/or mood disorders, schizophrenia and schizo-affective disorder, bipolar disorders, anxiety, anxio-depressive disorders, depression, mania, obsessive-compulsive disorders, impulse control disorders (e.g., Gilles de la Tourette's syndrome), attention disorders like ADD/ADHD, stress, and neurological disorders such as dementia and cognitive and/or memory dysfunction (e.g., amnesia, Alzheimer's disease, Pick's dementia, dementia of ageing, vascular dementia, mild cognitive impairment, age-related cognitive decline, and mild dementia of ageing), neurological and/or neurodegenerative disorders (e.g. Multiple Sclerosis, Raynaud's syndrome, Parkinson's disease, Huntington's chorea and Alzheimer's disease), demyelinisation-related disorders, neuroinflammatory disorders (e.g., Guillain-Barré syndrome).

In a further aspect the present invention provides the use of a compound of formula I in the preparation of a medicament for the treatment or prophylaxis of dependence and addictive disorders and behaviours (e.g., alcohol and/or drug abuse, pathological gambling, kleptomania), drug withdrawal disorders (e.g., alcohol withdrawal with or without perceptual disturbances; alcohol withdrawal delirium; amphetamine withdrawal; cocaine withdrawal; nicotine withdrawal; opioid withdrawal; sedative, hypnotic or anxiolytic withdrawal with or without perceptual disturbances; sedative, hypnotic or anxiolytic withdrawal delirium; and withdrawal symptoms due to other substances), alcohol and/or drug-induced mood, anxiety and/or sleep disorder with onset during withdrawal, and alcohol and/or drug relapse.

In a further aspect the present invention provides the use of a compound of formula I in the preparation of a medicament for the treatment or prophylaxis of neurological dysfunctions such as dystonias, dyskinesias, akathisia, tremor and spasticity, treatment of spinal cord injury, neuropathy, migraine, vigilance disorders, sleep disorders (e.g., disturbed sleep architecture, sleep apnea, obstructive sleep apnea, sleep apnea syndrome), pain disorders, cranial trauma.

In a further aspect the present invention provides the use of a compound of formula I in the preparation of a medicament for the treatment or prophylaxis of immune, cardiovascular disorders (e.g. atherosclerosis, arteriosclerosis, angina pectoris, abnormal heart rythms, and arrythmias, congestive heart failure, coronary artery disease, heart disease, hypertension, prevention and treatment of left ventricular hypertrophy, myocardial infarction, transient ischaemic attack, peripheral vascular disease, systemic inflammation of the vasculature, septic chock, stroke, cerebral apoplexy, cerebral infarction, cerebral ischaemia, cerebral thrombosis, cerebral embolism, cerebral hemorrhagia, metabolic disorders (e.g. conditions showing reduced metabolic activity or a decrease in resting energy expenditure as a percentage of total fat-free mass, diabetes mellitus, dyslipidemia, fatty liver, gout, hypercholesterolemia, hyperlipidemia, hypertriglyceridemia, hyperuricacidemia, impaired glucose tolerance, impaired fasting glucose, insulin resistance, insulin resistance syndrome, metabolic syndrome, syndrome X, obesity-hypoventilation syndrome (Pickwickian syndrome), type I diabetes, type II diabetes, low HDL- and/or high LDL-cholesterol levels, low adiponectin levels), reproductive and endocrine disorders (e.g. treatment of hypogonadism in males, treatment of infertility or as contraceptive, menstrual abnormalities/emmeniopathy, polycystic ovarian disease, sexual and reproductive dysfunction in women and men (erectile dysfunction), GH-deficient subjects, hirsutism in females, normal variant short stature) and diseases related to the respiratory (e.g. asthma and chronic obstructive pulmonary disease) and gastrointestinal systems (e.g. dysfunction of gastrointestinal motility or intestinal propulsion, diarrhea, emesis, nausea, gallbladder disease, cholelithiasis, obesity-related gastro-esophageal reflux, ulcers).

In a further aspect the present invention provides the use of a compound of formula I in the preparation of a medicament for the treatment or prophylaxis of dermatological disorders, cancers (e.g. colon, rectum, prostate, breast, ovary, endometrium, cervix, gallbladder, bile duct), craniopharyngioma, Prader-Willi syndrome, Turner syndrome, Frohlich's syndrome, glaucoma, infectious diseases, urinary tract disorders and inflammatory disoerders (e.g. arthritis deformans, inflammation, inflammatory sequelae of viral encephalitis, osteoarthritis) and orthopedic disorders.

In another aspect the present invention provides a compound of formula I as previously defined for use as a medicament.

In a further aspect the present invention provides the use of a compound of formula I in the preparation of a medicament for the treatment or prophylaxis of obesity, psychiatric disorders such as psychotic disorders, schizophrenia, bipolar disorders, anxiety, anxio-depressive disorders, depression, cognitive disorders, memory disorders, obsessive-compulsive disorders, anorexia, bulimia, attention disorders like ADHD, epilepsy, and related conditions, neurological disorders such as dementia, neurological disorders (e.g. Multiple Sclerosis), Parkinson's Disease, Huntington's Chorea and Alzheimer's Disease, immune, cardiovascular, reproductive and endocrine disorders, septic shock, diseases related to the respiratory and gastrointestinal systems (e.g. diarrhea), and extended abuse, addiction and/or relapse indications, e.g. treating drug (nicotine, ethanol, cocaine, opiates, etc) dependence and/or treating drug (nicotine, ethanol, cocaine, opiates, etc) withdrawal symptoms.

The compounds of the present invention are particulary suitable for the preparation of a medicament for the treatment of obesity, e.g. by reduction of appetite and body weight, maintenance of weight reduction and prevention of rebound.

The compounds of the present invention may also be used in the preparation of a medicament for preventing or reversing medication-induced weight gain, e.g. weight gain caused by antipsychotic (neuroleptic) treatment(s). The medicaments of the present invention may also be used to prevent or reverse weight gain associated with smoking cessation

### Combination Therapy

The compounds of the invention may be combined with another therapeutic agent that is useful in the treatment of obesity such as other anti-obesity drugs, that affect energy expenditure, glycolysis, gluconeogenesis, glucogenolysis, lipolysis, lipogenesis, fat absorption, fat storage, fat excretion, hunger and/or satiety and/or craving mechanisms, appetite/motivation, food intake, or G-I motility.

The compounds of the invention may further be combined with another therapeutic agent in the preparation of a medicament that is useful in the treatment of disorders associated with obesity such as hypertension, hyperlipidaemias, dyslipidaemias, diabetes, sleep apnea, asthma, heart disorders, atherosclerosis, macro and micro vascular diseases, liver steatosis, cancer, joint disorders, and gallbladder disorders. For example, a compound of the present invention may be used in combination with a another therapeutic agent that lowers blood pressure or that decreases the ratio of LDL:HDL or an agent that causes a decrease in circulating levels of LDL-cholesterol. In patients with diabetes mellitus the compounds of the invention may also be combined with therapeutic agents used to treat complications related to micro-angiopathies.

The pharmaceutical formulation of the invention may be used alongside other therapies for the treatment of obesity and its associated complications the metabolic syndrome and type 2 diabetes, these include biguanide drugs, insulin (synthetic insulin analogues) and oral antihyperglycemics (these are divided into prandial glucose regulators and alpha-glucosidase inhibitors).

In another aspect of the invention, the compound of formula I, or a pharmaceutically acceptable salt thereof may be administered in association with a PPAR modulating agent. PPAR modulating agents include but are not limited to a PPAR alpha and/or gamma agonist, or pharmaceutically acceptable salts, solvates, solvates of such salts or prodrugs thereof. Suitable PPAR alpha and/or gamma agonists, pharmaceutically acceptable salts, solvates, solvates of such salts or prodrugs thereof are well known in the art.

In addition the combination of the invention may be used in conjunction with a sulfonylurea. The present invention also includes a compound of the present invention in combination with a cholesterol-lowering agent. The cholesterol-lowering agents referred to in this application include but are not limited to inhibitors of HMG-CoA reductase (3-hydroxy-3-methylglutaryl coenzyme A reductase). Suitably the HMG-CoA reductase inhibitor is a statin.

In the present application, the term "cholesterol-lowering agent" also includes chemical modifications of the HMG-CoA reductase inhibitors, such as esters, prodrugs and metabolites, whether active or inactive.

The present invention also includes a compound of the present invention in combination with an inhibitor of the ileal bile acid transport system (IBAT inhibitor). The present invention also includes a compound of the present invention in combination with a bile acid binding resin.

The present invention also includes a compound of the present invention in combination with a bile acid sequestering agent, for example colestipol or cholestyramine or cholestagel.

According to an additional further aspect of the present invention there is provided a pharmaceutical formulation useful in a combination treatment comprising the administration of an effective amount of a compound of the formula I, or a pharmaceutically acceptable salt thereof, optionally together with a pharmaceutically acceptable diluent or carrier, with the simultaneous, sequential or separate administration one or more of the following agents selected from:
a CETP (cholesteryl ester transfer protein) inhibitor;
a cholesterol absorption antagonist;
a MTP (microsomal transfer protein) inhibitor ;
a nicotinic acid derivative, including slow release and combination products;
a phytosterol compound ;
probucol;
an anti-coagulant;
an omega-3 fatty acid ;
another anti-obesity compound for example sibutramine, phentermine, orlistat, bupropion, ephedrine, thyroxine;
an antihypertensive compound for example an angiotensin converting enzyme (ACE)
inhibitor, an angiotensin II receptor antagonist, an adrenergic blocker, an alpha adrenergic blocker, a beta adrenergic blocker, a mixed alpha/beta adrenergic blocker, an adrenergic stimulant, calcium channel blocker, an AT-1 blocker, a saluretic, a diuretic or a vasodilator;
a melanin concentrating hormone (MCH) modulator;
an NPY receptor modulator;
an orexin receptor modulator;
a phosphoinositide-dependent protein kinase (PDK) modulator; or
modulators of nuclear receptors for example LXR, FXR, RXR, GR, ERRα, β, PPARα, β, γ and RORalpha;
a monoamine transmission-modulating agent, for example a selective serotonin reuptake inhibitor (SSRI), a noradrenaline reuptake inhibitor (NARI), a noradrenaline-serotonin reuptake inhibitor (SNRI), a monoamine oxidase inhibitor (MAOI), a tricyclic antidepressive agent (TCA), a noradrenergic and specific serotonergic antidepressant (NaSSA);
an antipsychotic agent for example olanzapine and clozapine;
a serotonin receptor modulator;
a leptin/leptin receptor modulator;
a ghrelin/ghrelin receptor modulator;
a DPP-IV inhibitor;
or a pharmaceutically acceptable salt, solvate, solvate of such a salt or a prodrug thereof, optionally together with a pharmaceutically acceptable diluent or carrier to a warm-blooded animal, such as man in need of such therapeutic treatment.

According to an additional further aspect of the present invention there is provided a pharmaceutical formulation useful in a combination treatment comprising the administration of an effective amount of a compound of the formula I, or a pharmaceutically acceptable salt thereof, optionally together with a pharmaceutically acceptable diluent or carrier, with the simultaneous, sequential or separate administration of very low calorie diets (VLCD) or low-calorie diets (LCD).

Therefore in an additional feature of the invention, there is provided a compound of formula I for use in the treatment of obesity and its associated complications in a warm-blooded animal, such as man, in need of such treatment which comprises administering to said animal an effective amount of a compound of formula I, or a pharmaceutically acceptable salt thereof in simultaneous, sequential or separate administration with an effective amount of a compound from one of the other classes of compounds described in this combination section, or a pharmaceutically acceptable salt, solvate, solvate of such a salt or a prodrug thereof.

Therefore in an additional feature of the invention, there is provided a compound of formula I for use in treating hyperlipidemic conditions in a warm-blooded animal, such as man, in need of such treatment which comprises administering to said animal an effective amount of a compound of formula I, or a pharmaceutically acceptable salt thereof in simultaneous, sequential or separate administration with an effective amount of a compound from one of the other classes of compounds described in this combination section or a pharmaceutically acceptable salt, solvate, solvate of such a salt or a prodrug thereof.

According to a further aspect of the invention there is provided a pharmaceutical composition which comprises a compound of formula I, or a pharmaceutically acceptable salt thereof, and a compound from one of the other classes of compounds described in this combination section or a pharmaceutically acceptable salt, solvate, solvate of such a salt or a prodrug thereof, in association with a pharmaceutically acceptable diluent or carrier. According to a further aspect of the present invention there is provided a kit comprising a compound of formula I, or a pharmaceutically acceptable salt thereof, and a compound from one of the other classes of compounds described in this combination section or a pharmaceutically acceptable salt, solvate, solvate of such a salt or a prodrug thereof. According to a further aspect of the present invention there is provided a kit comprising:
a) a compound of formula I, or a pharmaceutically acceptable salt thereof, in a first unit dosage form;
b) a compound from one of the other classes of compounds described in this combination section or a pharmaceutically acceptable salt, solvate, solvate of such a salt or a prodrug thereof; in a second unit dosage form; and
c) container means for containing said first and second dosage forms.

According to a further aspect of the present invention there is provided a kit comprising:
a) a compound of formula I, or a pharmaceutically acceptable salt thereof, together with a pharmaceutically acceptable diluent or carrier, in a first unit dosage form;
b) a compound from one of the other classes of compounds described in this combination section or a pharmaceutically acceptable salt, solvate, solvate of such a salt or a prodrug thereof, in a second unit dosage form; and
c) container means for containing said first and second dosage forms.

According to another feature of the invention there is provided the use of a compound of the formula I, or a pharmaceutically acceptable salt thereof, and one of the other compounds described in this combination section, or a pharmaceutically acceptable salt, solvate, solvate of such a salt or a prodrug thereof, in the manufacture of a medicament for use in the the treatment of obesity and its associated complications in a warm-blooded animal, such as man.

According to another feature of the invention there is provided the use of a compound of the formula I, or a pharmaceutically acceptable salt thereof, and one of the other compounds described in this combination section, or a pharmaceutically acceptable salt, solvate, solvate of such a salt or a prodrug thereof, in the manufacture of a medicament for use in the treatment of hyperlipidaemic conditions in a warm-blooded animal, such as man. According to a further aspect of the present invention there is provided a combination treatment comprising the administration of an effective amount of a compound of the formula I, or a pharmaceutically acceptable salt thereof, optionally together with a pharmaceutically acceptable diluent or carrier, with the simultaneous, sequential or separate administration of an effective amount of one of the other compounds described in this combination section, or a pharmaceutically acceptable salt, solvate, solvate of such a salt or a prodrug thereof, optionally together with a pharmaceutically acceptable diluent or carrier to a warm-blooded animal, such as man in need of such therapeutic treatment. Furthermore, a compound of the invention may also be combined with therapeutic agents that are useful in the treatment of disorders or conditions associated with obesity (such as type II diabetes, metabolic syndrome, dyslipidemia, impaired glucose tolerance, hypertension, coronary heart disease, non-alcoholic steatohepatitis, osteoarthritis and some cancers) and psychiatric and neurological conditions.

It will be understood that there are medically accepted defmitions of obesity and being overweight. A patient may be identified by, for example, measuring body mass index (BMI), which is calculated by dividing weight in kilograms by height in metres squared, and comparing the result with the definitions.

### Pharmacological Activity

Compounds of the present invention are active against the receptor product of the CB1 gene. The affinity of the compounds of the invention for central cannabinoid receptors is demonstrable in methods described in Devane et al, Molecular Pharmacology, 1988, 34,605 or those described in WO01/70700 or EP 656354. Alternatively the assay may be performed as follows.

10µg of membranes prepared from cells stably transfected with the CB1 gene were suspended in 200µl of 100mM NaCl, 5mM MgCl₂, 1mM EDTA, 50mM HEPES (pH 7.4), 1mM DTT, 0.1% BSA and 100µM GDP. To this was added an EC80 concentration of agonist (CP55940), the required concentration of test compound and 0.1 µCi [³⁵S]-GTPγS. The reaction was allowed to proceed at 30°C for 45 min. Samples were then transferred on to GFB filters using a cell harvester and washed with wash buffer (50mM Tris (pH 7.4), 5mM MgCl₂, 50mM NaCl). Filters were then covered with scintilant and counted for the amount of [³⁵S]-GTPγS retained by the filter.

Activity is measured in the absence of all ligands (minimum activity) or in the presence of an EC80 concentration of CP55940 (maximum activity). These activities are set as 0% and 100% activity respectively. At various concentrations of novel ligand, activity is calculated as a percentage of the maximum activity and plotted. The data are fitted using the equation y=A+((B-A)/1+((C/x) ÙD)) and the IC50 value determined as the concentration required to give half maximal inhibition of GTPγS binding under the conditions used.

The compounds of the present invention are active at the CB1 receptor (IC50 <1 micromolar). Most preferred compounds have IC50 <200 nanomolar. For example the IC50 of Example 10 is 6.0nM and Example 11 is 6.4nM.

The compounds of the invention are believed to be selective CB1 antagonists or inverse agonists. The potency, selectivity profile and side effect propensity may limit the clinical usefulness of hitherto known compounds with alleged CB 1 antagonistic/inverse agonistic properties. In this regard, preclinical evaluation of compounds of the present invention in models of gastrointestinal and/or cardiovascular function indicates that they offer significant advantages compared to representative reference CB1 antagonist/inverse agonist agents.

The compounds of the present invention may provide additional benefits in terms of potency, selectivity profile, bioavailability, half-life in plasma, blood brain permeability, plasma protein binding ( for example higher free fraction of drug) or solubility compared to representative reference CB 1 antagonists/inverse agonist agents.

The utility of the compounds of the present invention in the treatment of obesity and related conditions is demonstrated by a decrease in body weight in cafeteria diet-induced obese mice. Female C57B1/6J mice were given ad libitum access to calorie-dense 'cafeteria' diet (soft chocolate/cocoa-type pastry, chocolate, fatty cheese and nougat) and standard lab chow for 8-10weeks. Compounds to be tested were then administered systemically (iv, ip, sc or po) once daily for a minimum of 5 days, and the body weights of the mice monitored on a daily basis. Simultaneous assessment of adiposity was carried by means of DEXA imaging at baseline and termination of the study. Blood sampling was also carried out to assay changes in obesity-related plasma markers.

### Examples

### Abbreviations

- AcOH: acetic acid
- DCM: dichloromethane
- DMF: dimethylformamide
- DEA: diethylamine
- DIEA: *N*,*N*-diisopropylethylamine
- DMAP: 4-dimethylaminopyridine
- EtOAc: ethyl acetate
- Et₃N: triethylamine
- Ex or EX: Example
- LiHMDS: lithium hexamethyldisilazide
- MeOH: methanol
- MeCN: acetnitrile
- rt or RT: room temperature
- TEA: Triethylamine
- THF: tetrahydrofuran
- t: triplet
- s: singlet
- d: doublet
- q: quartet
- qvint: quintet
- m: multiplet
- br: broad
- bs: broad singlet
- dm: doublet of multiplet
- bt: broad triplet
- dd: doublet of doublet

### General Experimental Procedures

Mass spectra were recorded on either a Micromass ZQ single quadrupole or a Micromass LCZ single quadrupole mass spectrometer both equipped with a pneumatically assisted electrospray interface (LC-MS). ¹H NMR measurements were performed on either a Varian Mercury 300 or a Varian Inova 500, operating at ¹H frequencies of 300 and 500 MHz respectively. Chemical shifts are given in ppm with CDCl₃ as internal standard. CDCl₃ is used as the solvent for NMR unless otherwise stated. Purification was performed on a semipreparative HPLC (High Performance Liquid Chromatography) with a mass triggered fraction collector, Shimadzu QP 8000 single quadrupole mass spectrometer equipped with 19 x 100 mm C8 column. The mobile phase used was, if nothing else is stated, acetonitrile and buffer (0.1 M ammonium acetate: acetonitrile 95:5).

For isolation of isomers, a Kromasil CN E9344 (250 x 20 mm i.d.) column was used. Heptane:ethyl acetate:DEA 95:5:0.1 was used as mobile phase (1 ml/min). Fraction collection was guided using a UV-detector (330 nm).

Typical HPLC-parameters for purity analysis:
HPLC-system: Agilent 1100
Column: Zorbax Eclipse XDB-C8 150x4.6 mm
Time of analysis: 15 min
Flow: 1.5ml/min
Mobilphase: A: water, 5% MeOH
   B: MeOH
Temperature: 40 °C
Detector; Uv 240nm

### Examples of the Invention

### Example 1

### Step A 1-(4-Benzyloxyphenyl)propan-1-one

4-Hydroxypropiophenone (15.0 g, 0.10 mol) was dissolved in acetone (200 ml) together with potassium carbonate (13.8 g, 0.10 mol). Benzyl bromide (17.1 g, 0.10 mol) was added and the reaction mixture heated at reflux overnight. After cooling to room temperature the mixture was filtered and concentrated on the rotary evaporator to afford 24.0 g (100%) of the title compound as a white solid

### Step B 1-(4-Benzyloxyphenyl)-2-bromopropan-1-one

1-(4-Benzyloxyphenyl)propan-1-one (4.80 g, 20.0 mmol) was suspended in acetic acid (25 ml) and cooled to 0 °C. Bromine (3.20 g, 20.0 mmol) was added dropwise and the reaction mixture stirred two hours at room temperature at which point the reaction mixture was a clear, yellow solution. After cooling, water (100 ml) was added and the product extracted with ether (2 x 100 ml). The combined organic extracts were washed with water, sodium hydrogen carbonate and brine. The organic phase was dried (Na₂SO₄), filtered and evaporated leaving the title compound as a pale yellow solid (6.17 g, 97%).

### Step C 2-[2-(4-Benzyloxy-phenyl-2-oxo-ethyl-3-oxo-butyric acid ethyl ester

A solution of sodium ethoxide was generated from sodium metal (0.53 g, 23.0 mmol) in 30 ml abs. ethanol. To this solution was added ethyl acetoacetate (3.00 g, 23.0 mmol) at 0°C. After 30 min. this solution was added to a solution of 1-(4-benzyloxyphenyl)-2-bromo-propan-1-one (6.17 g, 19.0 mmol) in ethanol: toluene (30 : 15 ml) and the reaction mixture stirred overnight. Acidic work-up with 1 M HCl, extraction with ethyl acetate (3 x), washing with brine, drying (Na₂SO₄); filtering and evaporation left a crude product purified by flash chromatography (hexane : EtOAc 95 : 5 - 70 : 30) affording 5.18 g of the title compound as a pale yellow oil.

### Step D 5-(4-Benzyloxyphenyl)-1-(2,4-dichlorophenyl)-4-phenyl-1H-pyrazole-3-carboxylic acid

A solution of sodium ethoxide was generated from sodium metal (0.19 g, 8.26 mmol) in 20 ml abs. ethanol. To this solution was added 2-[2-(4-benzyloxyphenyl)-2-oxo-ethyl]-3-oxo-butyric acid ethyl ester (2.13 g, 6.00 mmol) and the reaction mixture stirred at room temperature for 30 min. A previously prepared solution of 2,4-dichlorophenyldiazonium chloride (prepared from 2,4-dichloroaniline (1.19 g, 7.30 mmol) in 3 ml 24% HCl and sodium nitrite (0.52 g, 7.50 mmol) in 3 ml water at 0 °C) was added in 5 portions keeping the temperature below 5 °C. After stirring at room temperature for 2.5 hours water was added, and the product extracted with EtOAc (3 x). The combined organic extracts were dried (Na₂SO₄), filtered and evaporated. The residue was dissolved in ethanol (40 ml) and sodium hydroxide (0.80 g, 20.0 mmol) in 10 ml of water was added. After 2 hours boiling under reflux the reaction mixture was cooled, acidified with HCl and the product extracted with EtOAc (3 x). After washing, drying (Na₂SO₄), filtration and concentration, the residue was purified by flash chromtography (hexane : EtOAc 70:30 - 50:50) affording 1.84 g (68%) of the title compound as a pale yellow solid.

### Step E 5-(4-Benzyloxyphenyl)-1-(2,4-dichlorophenyl)-4-methyl-1H-pyrazole-3-carboxylic acid piperidin-1-ylamide

5-(4-Benzyloxyphenyl)-1-(2,4-dichlorophenyl)-4-methyl-1*H*-pyrazole-3-carboxylic acid (1.84 g, 4.07 mmol) was suspended in dichloromethane and a few drops of DMF added followed by the addition of oxalyl chloride (1.03 g, 8.14 mmol). The reaction mixture was refluxed for two hours. After cooling to room temperature, the solvent was removed and the crude acid chloride redissolved in dichloromethane and cooled to 0 °C. Triethylamine (1.15 ml, 8.20 mmol) was added followed by 1-aminopiperidine (0.5 ml, 4.50 mmol). The cooling bath was removed and the reaction mixture stirred at room temperature for 2 hours. Water was added and the product extracted with dichloromethane (3 x). The combined extracts were dried (Na₂SO₄), filtered and evaporated. Flash chromatography (hexane : EtOAc 80 : 20 - 70 : 30) afforded 1.13 g (52%) of the title compound as a solid.

### Step F 1-(2,4-Dichlorophenyl)-5-(4-hydroxyphenyl)-4-methyl-1H-pyrazole-3-carboxylic acid piperidin-1-yl amide

5-(4-Benzyloxyphenyl)-1-(2,4-dichlorophenyl)-4-methyl-1*H*-pyrazole-3-carboxylic acid piperidin-1-ylamide (1.00 g, 1.87 mmol) was dissolved in 25 ml of abs. ethanol together with 100 mg of palladium on charcoal (10% Pd). The reaction was hydrogenated with a balloon overnight. Filtration, concentration and flash chromatography (hexane : EtOAc 50 : 50 - EtOAc) afforded 0.83 g (100%) of the title compound as a solid.

### Step G Propane-1-sulfonic acid 4-[2-(2,4-dichlorophenyl)-4-methyl-5-(piperidin-1-ylcarbamoyl)2H-pyrazol-3-yl]-phenyl ester

1-(2,4-Dichlorophenyl)-5-(4-hydroxyphenyl)-4-methyl-1*H*-pyrazole-3-carboxylic acid piperidin-1-yl amide (222 mg, 0.50 mmol) was dissolved in dichloromethane (10 ml) and triethylamine (0.07 ml, 0.50 mmol) was added. Propanesulfonyl chloride (71 mg, 0.50 mmol) was added at 0 °C, the cooling bath removed and the reaction stirred at room temperature for 2 hours. Water was added and the product was extracted with dichloromethane, dried (Na₂SO₄), filtered and concentrated. Flash chromatography (hexane : EtOAc 70 : 30 - 50 :50) afforded a product that was recrystallised from hexane : EtOAc to give 135 mg (49%) of the the title compound as a white solid m.p. 190 °C.
¹H NMR(CDCl₃): δ 7.66 (1H, broad s), 7.44-7.17 (7 H, m), 3.25 (2H, t), 2.90 (4H, m), 2.39 (3H, s), 2.09-1.97 (2H, m), 1.78 (4H, m), 1.45 (2 H, m), 1.17 (3H, t)
MS m/z 573 (M+Na)

### Reference Example 2

### 1-(2,4-Dichlorophenyl)-4-methyl-5-[4,4,4-trifluorobutoxyphenyl]-1H-pyrazole-3-carboxylic acid piperidin-1-ylamide

1-(2,4-Dichlorophenyl)-5-(4-hydroxyphenyl)-4-methyl-1*H*-pyrazole-3-carboxylic acid piperidin-1-yl amide from **Ex 1, Step F** (250 mg, 0.56 mmol) was dissolved in acetone (10 ml) and potassium carbonate (77 mg, 0.56 mmol) was added followed by 1-iodo-4,4,4-trifluorobutane (140 mg, 0.56 mmol). The reaction mixture was boiled under reflux overnight, concentrated and purified by flash chromatography (hexane : EtOAc 70 : 30 - 60 : 40) afforded 130 mg (42%) of a white solid that was triturated with hexane : EtOAc 95 : 5 and filtered.
¹H NMR(CDCl₃): δ 7.63 (1,H, broad s), 7.43 (1H, m), 7.30 (2H, m), 7.10-7.00 (2H, m), 6.85-6.78 (2H, m), 4.05 (2H, t), 2.90 (4H, m), 2.40-2.19 (5H, s and m), 2.15-1.97 (2H, m), 1.78 (4H, m), 1.45 (2 H, m)
MS m/z 577 (M+Na). HPLC: 98.4%

### Example 3

### Butane-1-sulfonic acid 4-[2-(2,4-dichlorophenyl)-4-methyl-5-(-piperidin-1-ylcarbamoyl)-2H-pyrazol-3-yl]-phenyl ester

1-(2,4-Dichlorophenyl)-5-(4-hydroxy-phenyl)-4-methyl-1*H*-pyrazole-3-carboxylic acid piperidin-1-yl amide, prepared as in **Ex 1, Step F** (350 mg, 0.78 mmol) was dissolved in dichloromethane (10 ml) and triethylamine (0.11 ml, 0.78 mmol) was added. Butanesulfonyl chloride (0.12 g, 0.78 mmol) was added at 0 °C, the cooling bath removed and the reaction stirred at room temperature overnight. Water was added, the product extracted with dichloromethane, dried (Na₂SO₄), filtered and concentrated. Flash chromatography (hexane : EtOAc 70 : 30 - 50 :50) afforded a product that was recrystallised from hexane : EtOAc to give 200 mg (45%) of the the title compound as a solid.
¹H NMR(CDCl₃): δ 7.48-7.19 (8H, m), 3.29 (2H, m), 2.96 (4H, m), 2.41 (3H, s), 2.09-1.97 (2H, m), 1.81 (4H, m), 1.64-1.50 (4 H, m), 1.02 (3H, t)

### Example 4

### Propane-1-sulfonic acid 4-[2-(2,4-dichlorophenyl)-4-methyl-5-(morpholin-4-ylcarbamoyl)-2H-pyrazol-3-yl]phenyl ester

### Step A 5-(4-Benzyloxyphenyl)-1-(2,4-dichlorophenyl)-4-methyl-1H-pyrazole-3-carboxylic acid morpholin-4-ylamide

To a solution of 5-(4-benzyloxyphenyl)-1-(2,4-dichlorophenyl)-4-methyl-1*H*-pyrazole-3-carboxylic acid, prepared as in **Ex 1, Step D** (1.18 g, 2.6 mmol) in 25 ml CH₂Cl₂ was added 2 drops of DMF followed by oxalyl chloride (0.44 ml, 5.2 mmol). The mixture was boiled under reflux for 2 hours, cooled to room temperature and evaporated to dryness. The residue was dissolved in 25 ml CH₂Cl₂ and cooled to 0°C. Triethylamine (0.73 ml, 5.2 mmol) was added followed by 1-aminopiperidine (0.28 ml, 2.9 mmol) and the mixture was stirred at room temperature for 3 hours. Water (100 ml) was added and the mixture was extracted with CH₂Cl₂ (3x50 ml), dried (Na₂SO₄), filtered and concentrated. Flash chromatography (silica, hexane:EtOAc 1:2, EtOAc) afforded 215 mg (15%) of the title compound as a white solid.

### Step B 1-(2,4-Dichlorophenyl)-5-(4-hydroxyphenyl)-4-methyl-1H-pyrazole-3-carboxylic acid morpholin-4-ylamide

5-(4-Benzyloxyphenyl)-1-(2,4-dichlorophenyl)-4-methyl-1*H*-pyrazole-3-carboxylic acid morpholin-4-ylamide (215 mg, 0.40 mmol) was dissolved in 20 ml CH₂Cl₂ and cooled to 0°C. Boron tribromide (78 µl, 0.80 mmol) was added dropwise and the reaction mixture was stirred at room temperature for 2.5 hours. Water (50 ml) was added and the solution extracted with EtOAc (3x50 ml). The combined organic phases were dried (Na₂SO₄), filtered and concentrated. Flash chromatography (silica, hexane:EtOAc 1:2, EtOAc) afforded 180 mg (99%) of the title compound as a white solid.

### Step C Propane-1-sulfonic acid 4-[2-(2,4-dichlorophenyl)-4-methyl-5-(morpholin-4-ylcarbamoyl)-2H-pyrazol-3-yl]-phen ester

A solution of 1-(2,4-dichlorophenyl)-5-(4-hydroxyphenyl)-4-methyl-1*H*-pyrazole-3-carboxylic acid morpholin-4-ylamide (180 mg, 0.40 mmol) in 10 ml CH₂Cl₂ was cooled to 0°C. Triethylamine (56 µl, 0.40 mmol) was added followed by 1-propanesulfonyl chloride (45 µl, 0.40 mmol) and the reaction mixture was stirred at room temperature for 5 hours.

Water was added and the mixture was extracted with CH₂Cl₂ (3x20 ml), dried (Na₂SO₄), filtered and concentrated. Flash chromatography (silica, hexane:EtOAc 1:2) afforded 82 mg (46%) of the title compound as a white solid.
¹HNMR (CDC13): δ 7.7 (1H, s), 7.5-7.4 (1H, m), 7.4-7.1 (6H, m), 3.9-3.8 (4H, m), 3.3-3.2 (2H, m), 3.0-2.9 (4H, m), 2.4 (3H, s), 2.1-1.9 (2H, m), 1.2 (3H, t).
MS m/z 576 (M+Na). HPLC: 98.0%.

### Example 5

### 3,3,3-Trifluoropropane-1-sulfonic acid 4-[2-(2,4-dichlorophenyl-4-methyl-5-(piperidin-1-ylcarbamoyl)-2H-pyrazol-3-yl]phenyl ester

### Step A 1-(2,4-Dichlorophenyl)-5-(4-hydroxyphenyl)-4-methyl-1H-pyrazole-3-carboxylic acid piperidin-1-yl amide

5-(4-Benzyloxyphenyl)-1-(2,4-dichlorophenyl)-4-methyl-1*H*-pyrazole-3-carboxylic acid piperidin-1-ylamide, prepared as in **Ex. 1, Step E** (330 mg, 0.62 mmol) was dissolved in 20 ml CH₂Cl₂ and cooled to 0°C. Boron tribromide (120 µl, 1.24 mmol) was added dropwise and the reaction mixture was stirred at room temperature for 1 hour. Water (50 ml) was added and the solution extracted with EtOAc (3x20 ml). The combined organic phases were dried (Na₂SO₄), filtered and concentrated. Flash chromatography (silica, hexane:EtOAc 1:3, EtOAc) afforded 130 mg (47%) of the title compound as a white solid.

### Step B 3,3,3-Trifluoropropane-1-sulfonic acid 4-[2-(2,4-dichlorophenyl)-4-methyl-5-(piperidin-1-ylcarbamoyl)-2H-pyrazol-3-yl]phenyl ester

A solution of 1-(2,4-dichlorophenyl)-5-(4-hydroxyphenyl)-4-methyl-1*H*-pyrazole-3-carboxylic acid piperidin-1-yl amide,(130 mg, 0.30 mmol) in 10 ml CH₂Cl₂ was cooled to 0°C. Triethylamine (42 µl, 0.30 mmol) was added followed by 3,3,3-trifluoropropane-1-sulfonyl chloride (59 mg, 0.30 mmol), purchased from Manchester Organics (but may also be prepared in an analogous manner to the method described in WO200010968 for 4,4,4-trifluorobutane-1-sulfonyl chloride), and the reaction mixture was stirred at room temperature for 2 hours, Water was added and the mixture was extracted with CH₂Cl₂ (3x20 ml), dried (Na₂SO₄), filtered and concentrated. Flash chromatography (silica, hexane:EtOAc 7:3, 6:4) afforded 150 mg (82%) of the title compound as a white solid m.p. 160 °C.
¹H NMR (CDCl₃): δ 7.7 (1H, broad s), 7.5-7.2 (7H, m), 3.6-3.5 (2H, m), 3.0-2.7 (6H, m), 2.4 (3H, s), 1.9-1.7 (4H, m), 1.6-1.4 (2H, m).
MS m/z 628 (M+Na). HPLC: 92.5%.

### Example 6

### 4 4,4-Trifluorobutane-1-sulfonic acid 4-[2-(2,4-dichlophenyl)-4-methyl-5-(piperidin-1-ylcarbamoyl)-2H-pyrazol-3-ylphenyl ester

1-(2,4-Dichlorophenyl)-5-(4-hydroxyphenl)-4-methyl-1*H-*pyrazole-3-carboxylic acid piperidin-1-yl amide, prepared as in **Ex 5, Step A** (0.49 g, 1.20 mmol) was dissolved in dichloromethane (20 ml), cooled to 0°C and triethylamine (0.67 ml, 4.8 mmol) added followed by 4,4,4-trifluorobutane-1-sulfonyl chloride, prepared as described in W0200010968, (0.38 g, 1.80 mmol). The reaction mixture was stirred at room temperature overnight. Water was added, the product extracted with dichloromethane, dried (Na₂SO₄), filtered and concentrated. Flash chromatography (hexane : EtOAc 1:1 - EtOAc) followed by recrystallisation (hexane : EtOAc) afforded 0.32 g (43%) of the title compound as a colorless solid.
¹H NMR(CDCl₃): δ 7.80 (1H, broad s), 7.50-7.19 (7H, m), 3.40 (2H, m), 3.05-2.90 (4H, m), 2.50-2.20 (7H, s and m), 1.92-1.70 (4H, m), 1.57-1,40 (2H, m).
MS m/z 641 (M+Na)
HPLC: 96.5%

### Example 7

### Propane-1-sulfonic acid-4-[2-(2,4-dichlorophenyl)-4-methyl-5-(piperidin-1-ylcarbamoyl)-2H-pyrazol-3-yl]-2,6-difluoropbenyl ester

### Step A 1-(4-Benzyloxy-3,5-difluorophenyl)-propane-1-one

1-(3,5-Difluoro-4-hydroxyphenyl)propane-1-one (5.00 g, 26,9 mmol) was dissolved in acetone (100 ml) together with potassium carbonate (3.90 g, 28.2 mmol). Benzyl bromide (4.82 g, 28.2 mmol) was added and the reaction mixture was boiled under reflux overnight. After cooling to room temperature the mixture was filtered and concentrated on a rotary evaporator to afford 7.43 g (100%) of the title compound as a white solid.

### Step B 1-(4-Benzyloxy-3,5-difluorophenyl)-2-bromopropan-1-one

1-(4-Benzyloxy-3,5-difluorophenyl)propane-1-one (7.43 g, 26.9 mmol) was suspended in acetic acid (35 ml). Bromine (4.28 g, 26.8 mmol) was added dropwise and the reaction mixture stirred two hours at room temperature at which point the reaction mixture was a clear, yellow solution. After cooling, ice-water (100 ml) was added and the product extracted with ether (2 x 100 ml). The combined organic extracts were washed with water, sodium hydrogen carbonate solution and brine. The organic phase was dried (Na₂SO₄), filtered and evaporated leaving 9.30 g (98%) of the title compound as a pale yellow oil.

### Step C 2-Acetyl-4-benzyloxy-3,5-difluorophenyl)-3-methyl-4-oxobutyric acid ethyl ester

A solution of sodium ethoxide was generated from sodium metal (0.74 g, 32.0 mmol) in 40 ml absolute ethanol. To this solution was added ethyl acetoacetate (4.16 g, 32.0 mmol) at 0 °C. After 30 min. this solution was added to a solution of 1-(4-benzyloxy-3,5-difluorophenyl)-2-bromopropan-1-one (9.30 g, 26.2 mmol) in ethanol: toluene (40 : 20 ml) and the reaction mixture stirred overnight. Acidic work-up with 1 M HCl, extraction with ethyl acetate (3 x), washing with brine, drying (Na₂SO₄), filtering and evaporation left a crude product purified by flash chromatography (hexane : EtOAc 95 : 5 - 70 : 30) affording 6.95 g (66%) of the title compound as an oil.

### Step D 5-(4-Benzyloxy-3,5-difluorophenyl)-1-(2,4-dichlorophenyl)-4-methyl-1H-pyrazole-3-carboxylic acid

A solution of sodium ethoxide was generated from sodium metal (0.53 g, 22.0 mmol) in 60 ml absolute ethanol. To this solution was added 2-acetyl-4-(4-benzyloxy-3,5-difluorophenyl)-3-methyl-4-oxobutyric acid ethyl ester (6.95 g, 17.2 mmol) and the reaction mixture stirred at room temperature for 30 min. A previously prepared solution of 2,4-dichlorophenyldiazonium chloride (prepared from 2,4-dichloroaniline (3.39 g, 21.0 mmol) in 9 ml 24% HCl and sodium nitrite 1. (48 g, 21.0 mmol) in 3 ml water at 0 °C) was added in 5 portions keeping the temperature below 5 °C. After stirring at 0 °C for 2 hours the reaction mixture was allowed to reach room temperature and stirred overnight. Water was added, the product extracted with EtOAc (3 x). The combined organic extracts were dried (Na₂SO₄), filtered and evaporated leaving 9.20 g of the crude ethyl ester as an oil.

The residue (9,20 g) was dissolved in ethanol (120 ml) and sodium hydroxide (2.30 g, 57.5 mmol) in 15 m1 of water was added. After 2 hours of boiling under reflux the reaction mixture was cooled, acidified with HCl and the product extracted with EtOAc (3 x). After washing, drying (Na₂SO₄), filtration and concentration, the residue was purified by flash chromtography (hexane : EtOAc : AcOH 80:20:2 - hexane : EtOAc : AcOH 50;50;2) affording 5.46 g (65%, two steps) of the title compound as a solid.

### Step E 5-(4-Benzyloxy-3,5-difluorohenyl)-1-(2,4-dichlorophenyl-)-4-methyl-1H-pyrazole-3-carboxylic acid piperidin-1-ylamide

5-(4-Benzyloxy-3,5-difluorophenyl)-1-(2,4-dichlorophenyl)-4-methyl-1*H-*pyrazole-3-carboxylic acid (5.46 g, 11.2 mmol) was suspended in dichloromethane (60 ml) and a few drops of DMF added followed by the addition of oxalyl chloride (4,70 ml, 55.8 mmol). The reaction mixture was boiled under reflux for 1,5 hours. After cooling to room temperature the solvent was removed and the crude acid chloride redissolved in dichloromethane, cooled to 0 °C, Et₃N (3.10 ml, 22.2 mmol) was added followed by 1-aminopiperidine (1.2 ml, 11.2 mmol). The cooling bath was removed and the reaction mixture stirred at room temperature overnight. Water was added and the product extracted with dichloromethane (3 x), the combined extracts dried (Na₂SO₄), filtered and evaporated. Flash chromatography (hexane : EtOAc 80 : 20-70: 30) afforded 1.86 g (30%) of the title compound as a yellow solid,

### Step F 5-(4-Hydroxy-3,5-difluorophenyl)-1-(2,4-dichlorophenyl)1-4-methyl-1H-pyrazole-3-carboxylic acid piperidin-1-ylamide

5-(4-Benzyloxy-3,5-difluorophenyl)-1-(2,4-dichlorophenyl)-4-methyl-1*H*-pyrazole-3-carboxylic acid piperidin-1-ylamide (1.86 g, 3.25 mmol) was dissolved in 50 ml of dichloromethane and cooled to -78 °C. BBr₃ (0.60 ml, 6.50 mmol) was added slowly and the reaction mixture stirred at 0°C for 30 min. Water was added and the product extracted with CH₂Cl₂ (x3). The combined organic extracts were dried (Na₂SO₄), filtered and concentrated, Flash chromatography (hexane : EtOAc 50 : 50) afforded 0.64 g (41%) of the title compound as a pale yellow solid.

### Step G Propane-1-sulfonic acid-4 -[2-(2,4-dicchloropheny)-4-methyl-5(piperidin-1-ylcarbamoyl)-2H-pyrazol-3-yl]-2,6-difluorphenyl ester

5-(4-Hydroxy-3,5-difluorophenyl)-1-(2,4-dichlorophenyl)-4-methyl-1*H*-pyrazole-3-carboxylic acid piperidin-1-ylamide (0.64 g, 1.32 mmol) was dissolved in dichloromethane (20 ml), cooled to 0°C and triethylamine (0.18 ml, 1.32 mmol) added followed by propanesulfonyl chloride (0.19 g, 1.31 mmol). The reaction mixture was stirred at room temperature overnight. Water was added, the product extracted with dichloromethane, dried (Na₂SO₄), filtered and concentrated. Flash chromatography (hexane : EtOAc 70 : 30 -50 : 50) afforded 410 mg (53%) of the title compound as pale yellow solid.
¹H NMR(CDCl₃): δ 7.66 (1H, broad s), 7.60-7.24 (4 H, m), 6.97-6.78 (1H, m), 3.50-3.37 (2H, m), 3.02-2.80 (4H, m), 2.40 (3 H, s), 2.20-2.00 (2H, m), 1.92-2.72 (4 H, m), 1.60-1.40 (2 H, m), 1.08 (3H, t).
MS m/z 609 (M+Na). HPLC: 97.5%.

### Example 8

### Propane-1-sulfonic acid 4-[2-(2,4-dichlorophen)-5-(piperidin-1-ylcarbamoyl)-2H-pyrazol-3-yl]phenyl ester

### Step A 4-(4-Benzyloxyphenyl)-2,4-dioxobutyric acid ethyl ester

To a solution of LiHMDS (88 ml, 1M in THF) in ether (50 ml) under nitrogen at -78 °C was added during 1 hour a suspension of 1-(4-benzyloxyphenyl)ethanone (20 g, 88.4 mmol) dissolved in ether (150 ml) and THF (50 ml). The resulting mixture was stirred at - 78 °C for 1 hour and then oxalic acid diethyl ester (14.2 g, 97.2 mmol) was added. The resulting mixture was slowly warmed to room temperature and then left overnight. The reaction mixture was diluted with pentane (90 ml) and the crude product precipitated as its lithium salt. The collected solid (27.2 g) was dried under vacuum and used directly in the next step.

### Step B 5-(4-Benzyloxyphenyl)-1-(2,4-dichlorophenyl)-1H-pyrazole-3-carboxylic acid ethyl ester

4-(4-Benzyloxyphenyl)-2,4-dioxobutyric acid ethyl ester (27.2 g, as Li salt from previous step) was suspended in ethanol (350 ml) and 2,4-dichlorophenylhydrazine (17.8 g, 83.3 mmol) was added. The reaction mixture was stirred overnight at room temperature. The solvent was then removed under reduced pressure and the residue was dissolved in acetic acid and the resulting mixture was refluxed for 24 h. The reaction mixture was diluted with ethyl acetate (1L) and then washed with saturated NaHCO₃ (6x250 ml) and brine (100 ml). The organic layer was dried (MgSO₄), filtered and concentrated under reduced pressure to give an oil. The oil was purified by flash chromatography (SiO₂, 20% EtOAc in heptane). The product fraction was concentrated under reduced pressure and the remaining material was then further purified by recrystallisation (ethyl acetate/heptane) to give a white solid (19.6, 57% over 2 steps).
¹H-NMR (CDCl₃): δ 1.42 (t, 3H), 4.45 (q, 2H), 5.03 (s, 2H), 6.88 (d, 2H), 7.01 (s, 1H), . 7.11 (d,2H), 7.3-7.45 (m, 8H).
MS: 467 (M+1).

### Step C 1-(2,4-Dichlorophenyl)-5-(4-hydroxyphenyl)-1H-pyrazole-3-carboxylic acid ethyl ester

To a solution of 5-(4-benzyloxyphenyl)-1-(2,4-dichlorophenyl)-1*H*-pyrazole-3-carboxylic acid ethyl ester (735 mg, 1.57 mmol) and (CH₃)₂S (0.58 ml, 7.86 mmol) in dichloromethane (30 ml) under nitrogen was added BF₃-diethyl etherate (1.0 ml, 7.86 mmol) dropwise. The resulting mixture was stirred overnight at room temperature. More (CH₃)₂S (0.58 ml, 7.86 mmol) and BF₃-diethyl etherate (1.0 ml, 7.86 mmol) was then added and the resulting mixture was stirred for another 3 days. The reaction mixture was diluted with dichloromethane to 80 ml and washed with water (3x30 ml) and brine (40 ml). The organic layer was dried (MgSO₄), filtered and concentrated under reduced pressure to give a white solid (573 mg, 96%). The crude material was used directly.
¹H-NMR (CDCl₃): δ 1.41 (t, 3H), 4.44 (q, 2H), 6.74 (d, 2H), 7.00 (s, 1H), 7.06 (d, 2H), 7.3-7.45 (m, 3H). MS: 375 (M-1).

### Step D 1-(2,4-Dichlorophenyl)-5-[4-(propane-1-sulfonyloxy)-phenyl]-1H-pyrazole-3-carboxylic acid ethyl ester

1-(2,4-Dichlorophenyl)-5-(4-hydroxyphenyl)-1*H*-pyrazole-3-carboxylic acid ethyl ester (510 mg, 1.35 mmol) was suspended in dichloromethane (20 ml) under nitrogen and triethylamine (0.75 ml, 5.4 mmol) was added. The resulting mixture was cooled to 0 °C and 1-propanesulfonyl chloride (0.30 ml, 2.7 mmol) was added dropwise. The mixture was stirred at 0 °C for 1 hour. The reaction mixture was then diluted to 40 ml with dichloromethane and then washed with saturated NaHCO₃ (3x20 ml) and brine (20 ml).

The organic layer was dried (MgSO₄), filtered and concentrated under reduced pressure to give an oil (0.64 g, 98%). The crude material was used as such without further purification. ¹H-NMR (CDCl₃): δ 1.12 (t, 3H), 1.43 (t, 3H), 2.01 (m, 2H), 3.23 (m, 2H), 4.46 (q, 2H), 7.07 (s,1H), 7.19-7.46 (m, 7H).
MS: 483 (M+1).

### Step E Propane-1-sulfonic acid 4-[2-(2,4-dichlorophenyl)-5-(piperidin-1)-yl(carbamoyl)-2H-pyrazol-3-yl]phenyl ester

1-Aminopiperidine hydrochloride (36 mg, 0.26 mmol) was dissolved in toluene (1.0 ml) under nitrogen atmosphere. Trimethylaluminium (2 M in toluene, 0.17 ml) was added dropwise at room temperature. The resulting mixture was then stirred at room temperature for 40 minutes. This mixture was then added to a stirred suspension of 1-(2,4-dichlorophenyl)-5-[4-(propane-1-sulfonyloxy)phenyl]-1*H*-pyrazole-3-carboxylic acid ethyl ester (42 mg, 0.087 mmol) in DCM (1.0 ml) and the resulting mixture was heated at 60 °C overnight. The reaction was quenched by addition of water and then partitioned between water (20 ml) and DCM (20 ml). The organic layer was washed with water (3x10 ml) and then concentrated under reduced pressure. The residue was purified by reverse phase HPLC, C8 column, 5-100% acetonitrile in water (buffer: 0.1 M ammonium acetate). The product fraction was diluted with ethyl acetate and washed with water several times. The organic layer was concentrated under reduced pressure and the residue was freeze-dried to give a white solid (26 mg, 55%).
¹H-NMR (CDCl₃): 1.11 (t, 3H), 1.43 (m, 2H), 1.76 (m, 4H), 2.00 (m, 2H), 2.85 (m, 4H), 3.22 (m, 2H), 7.11 (m, 1 H), 7.20 (m, 4H), 7.37 (m, 2H), 7.49 (m, 1H).
MS: 537 (M+1).

### Example 9

### Propane-1-sulfonic acid 4-[4-bromo-2-(2,4-dichlorophenl)-5-(piperidin-1-ylcarbamoyl)-2H-pyrazol-3-yl]phenyl ester

### Step A 4-Bromo-1-(2,4-dichlorophenyl)-5-[4-(propane-1-sulfonyloxy)phenyl]-1H-pyrazole-3-carboxylic acid ethyl ester

1-(2,4-Dichlorophenyl)-5-[4-(propane-1-sulfonyloxy)phenyl]-1H-pyrazole-3-carboxylic acid ethyl ester, prepared as in **Ex. 8, Step D** (597 mg, 1.23 mmol) was dissolved in dichloromethane (15 ml) and bromine (0.06 ml, 1.23 mmol) in dichloromethane (1 ml) was added and the resulting mixture was stirred at room temperature overnight. Extra bromine (0.06 ml, 1.23 mmol) was added and the mixture was stirred for another 20 hours.

The reaction mixture was diluted to 80 ml with dichloromethane and was then washed with saturated NaHCO₃ (40 ml), 20% Na₂S₂O₅ (40 ml), saturated NaHCO₃ (2x40 ml) and brine (40 ml). The organic layer was dried (MgSO₄), filtered and concentrated under reduced pressure to give an orange oil (0.598, 73%). The crude material was used without further purification. ¹H-NMR (CDCl₃): δ 1.12 (t, 3H), 1.44 (t, 3H), 2.01 (m, 2H), 3.24 (m, 2H), 4.48 (q, 2H), 7.2-7.46 (m, 7H). MS: 561

### Step B Propane-1-sulfonic acid 4-[4-bromo-2-(2,4-dichlorophenyl)-5-(piperidin-1-ylcarbamoyl)-2H-pyrazol-3-yl]phenyl ester

This compound was prepared in a similar manner to that described in **Ex.8 Step E.** by reacting 4-bromo-1-(2,4-dichlorophenyl)-5-[4-(propane-1-sulfonyloxy)phenyl]-1*H-*pyrazole-3-carboxylic acid ethyl ester with 1-aminopiperidine hydrochloride to give 26mg of the title compound as a white solid. Yield: 25%
¹H-NMR (CDCl₃): 1.12 (t, 3H), 1.43 (m, 2H), 1.74 (m, 4H), 2.01 (m, 2H), 2.90 (m, 4H), 3.24 (m, 2H), 7.21-7.45 (m, 7H)
MS: 615.

### Example 10

### Methyl 1-{[(1-(2,4-dichlorophenyl)-4-methyl-5-{4[(propylsulfonyl)oxy]phenyl}-1H-pyrazol-3-yl)carbonyl]amino}cyclopentanecarboxylate

### Step A Methyl 1-aminocyclopentanecabocylate hydrochloride

Thionyl chloride (1.5 ml) was dissolved in methanol (15 ml) and poured over 1-aminocyclopentanecarboxylic acid (100 mg, 0.774 mmol). The mixture was refluxed 1 hour. The solvent was evaporated to give the product (107 mg, 77%).
¹H NMR (399.964 MHz) δ 9.00-8.60 (br, 3H), 3.79 (s, 3H), 2.23 (s, 4H), 2.14-2.00 (m, 2H), 1.90-1.76 (m, 2H).

### Step B Methyl 1-({[5-[4-(benzyloxy)phenyl]-1-(2,4-dichlorophenyl)-4-methyl-1H-pyrazol-3-yl]carbonyl}amino)cyclopentanecarboxylate

A solution of 5-[4-benzyloxy)phenyl]-1-(2,4-dichlorophenyl)-4-methyl-1*H*-pyrazol-3-carboxylic acid, prepared as in **Ex.1, Step D** (59 mg, 0.130 mmol) in DCM (2 ml) was mixed with a solution of oxalyl chloride (2 ml) in DCM (20 ml). One drop of DMF was added and the reaction continued at room temperature in the dark for1 hour. The solvent was evaporated, DCM (2 ml) was added and the acid chloride mixture added to a mixture of methyl 1-aminocyclopentanecarboxylate hydrochloride (23 mg, 0.130 mmol) in DCM (2 ml) and K₂CO₃ (aq, 10 wt%, 2 ml). The reaction was continued at room temperature for 3 hours. The phases were separated and the organic phase washed with water and dried over MgSO₄ to give the product (71 mg, 94 %).
¹H NMR (399.964 MHz) δ 7.43-7.23 (m, 9H), 7.06-7.00 (m, 2H), 6.93-6.87 (m, 2H), 5.02 (s, 2H), 3.75 (s, 3H), 2.40-2.30 (m, 2H), 2.34 (s, 3H), 2.14-2.04 (m, 2H), 1.87-1.77 (m, 4H).
MS *m*/*z* 578, 580, 582 (M+H)⁺.

### Step C Methyl 1-({[1-(2,4-dichlorophenyl)-5-(4-hydroxyphenyl)-4-methyl-1H-pyrazol-3-yl]carbonyl}amino)cyclopentanecarboxylate

Boron trifluoride etherate (156 µl, 1.23 mmol) was added to a mixture of methyl 1-({[5-[4-(benzyloxy)phenyl]-1-(2,4-dichlorophenyl)-4-methyl-1*H*-pyrazol-3-yl]carbonyl}amino)cyclopentanecarboxylate (51 mg, 0.088 mmol) and dimethyl sulfide (90 µl, 1.23 mmol) in DCM (2 ml). The reaction was continued at room temperature in the dark for 46 hours. Water and DCM were added, the phases separated and the organic phase washed with water and dried over MgSO₄ (39 mg, 90%).
¹H NMR (399.964 MHz) δ 7.60-6.60 (m, 8H), 3.72 (s, 3H), 2.43-2.23 (m, 2H), 2.26 (s, 3H), 2.15-2.00 (m, 2H), 1.85-1.75 (m,4H).
MS *m*/*z* 488, 490, 492 (M+H)⁺.

### Step D Methyl 1-{[(1-(2,4-dichlorophenyl)-4-methyl-5-{4[(propylsulfonyl)oxy]phenyl}-1H-pyrazol-3-yl-carbonyl]amino}cyclopentanecarboxylate.

TEA (100 µl) and then 1-propanesulfonyl chloride (30 µl, 0.268 mmol) were added to a mixture of methyl 1-({[1-(2,4-dichlorophenyl)-5-(4-hydroxyphenyl)-4-methyl-1*H*-pyrazol-3-yl]carbonyl}amino)cyclopentanecarboxylate (39 mg, 0.080 mmol) in dry DCM (1.5 ml) at -78°C. The reaction was continued at -78°C under N₂(g) for 1.5 hours. Water and DCM were added and the temperature was raised to room temperature. The phases were separated and the orgainc phase washed with water and dried over MgSO₄. The product was purified by preparatory HPLC (kromasil C8 column, ammonium acetate (aq, 0.1 M):acetonitrile, product came at about 88% acetonitrile) to give the product as an almost white powder (17 mg, 35%).
¹H NMR (399.964 MHz) δ 7.45-7,39 (br, 1H), 7.32-7.28 (m, 2H), 7,27-7.19 (m, 3H), 7.18-7.12 (m, 2H), 3.76 (s, 3H), 3.26-3.20 (m, 2H), 2.40-2.30 (m, 2H), 2.35 (s, 3H), 2.15-2.05 (m, 2H), 2.05-1.95 (m, 2H), 1-88-1.78 (m, 4H), 1.12 (t, 3H).
HRMS Calcd for [C₂₇H₂₉Cl₂N₃O₆S+H]⁺; 594.123, Found: 594:121.

### Reference Example 11

### Carbonic acid 4-[2-(2,4-dichloro-phenyl)-4-methyl-5-(piperidine-1-ylcarbamoyl)-2H-pyrazol-3-yl-phenyl ester propyl ester

### Carbonic acid 4-[2-(2,4-dichlorophenyl)-4-methyl-5-(piperidine-1-ylcarbamoyl)-2H-pyrazol-3-yl[phenylester propyl ester

1-(2,4-Dichloro-phenyl)-5-(4-hydroxyphenyl)-4-methyl-1*H*-pyrazole-3-carboxylic acid piperidin-1-yl amide prepared as in Ex 1, Step F (0.44 g, 1.00 mmol) was dissolved in dichloromethane (10 ml) and triethylamine (0.28 ml, 2,24 mmol) was added. Propyl chloroformate (0.1.4 ml, 1.24 mmol) was added at 0 °C, and the reaction stirred for 40 min., concentrated and the product purified by flash chromatography (hexane : EtOAc 70 :30 - 50 : 50) to afford 345 mg (65%) of the title compound. Further purification by preparative HPLC, gave 239 mg of the title compound.
¹H NMR(CDCl₃): δ 7.71-7.18 (8H, m), 4.24 (2H, t), 2.93 (4H, m), 2.40 (3H, s), 1.78 (6H, m), 1.46 (2H, m), 1,03 (3H, t)
MS m/z 553 (M+Na)
HPLC; 94.15%

### Reference Example 12

### 4-{1-(2,4-dichlorophenyl)-4-methyl-3-[(piperidin-1-ylamino)carbonyl]-1H-pyrazol-5-yl}phenyl thiophene-2-sulfonate

Thiophene-2-sulfonyl chloride (433 mg, 2.37 mmol) in DCM (2.5 ml) was added to a mixture of 1-(2,4-dichlorophenyl)-5-(4-hydroxyphenyl)-4-methyl-*N*-piperidin-1-yl-1*H-*pyrazole-3-carboxamide prepared as in Ex 1, Step F (200 mg, 0.45 mmol) and TEA (0,5 ml, 3.59 mmol) in DCM (2.5 ml) at -78°C, under N₂(g). The reaction was continued at - 78°C for 2 hours and then at room temperature for 19 hours. Water was added and the phases were separated. The organic phase was washed with water and dried over MgSO₄. The product was further purified by preparatory HPLC (kromasil C8 column, ammonium acetate (aq, 0.1 M):acetonitrile, product came at 100% acetonitrile) to give an almost white powder (158 mg, 60%).
¹H NMR (399.964 MHz) δ 7.71-7.67 (m, 1H), 7.67-7.57 (br, 1H), 7.50-7.46 (m, 1H), 7.35 (s, 1H), 7.25 (s, 2H), 7,07,7.00.(m, 3H), 6.98-6.92 (m, 2H), 2.86-2.76 (m, 4H), 2.29 (s, 3H), 1.73-1.65 (m, 4H), 1.42-1.33 (m, 2H).
HRMS Calcd for [C₂₆H₂₄Cl₂N₄O₄S₂+H]⁺: 591.069. Found: 591.067.

### Reference Example 13

### 4-{1-(2,4-dichlorophenyl)-4-methyl-3[(piperidin-1-ylamino)carbonyl]-1H-pyrazol-5-yl}phenyl pyridine-3-sulfonate hydrochloride

### Step A: 4-{1-(2,4-dichlorophenyl)-4-methyl-3[(piperidin-1-ylamino)carbonyl]-1H-pyrazol-5-yl}phenyl pyridine-3-sulfonate

A suspension of pyridine-3-sulfonyl chloride (144 mg, 0.67 mmol) in DCM (10 ml) was added to a mixture of 1-(2,4-dichlorophenyl)-5-(4-hydroxyphenyl)-4-methyl-*N*-piperidin-1-yl-1*H-*pyrazole-3-carboxamide, prepared as Ex 1, Step F (200 mg, 0.45 mmol) and TEA (0,5 ml, 3.59 mmol) in DCM (2.5 ml) at -78°C, under N₂(g). The reaction was continued at -78°C for 1.5 hours and then at room temperature for 30 minutes. Water was added and the phases were separated. The organic phase was washed with water and dried over MgSO₄. The product was further purified by flash chromatography (SiO₂, toluene:ethyl acetate, product came at 42% ethyl acetate) to give an almost white powder (216 mg, 82%).
¹H NMR (399.964 MHz) δ 8.95-8.85 (m, 2H), 8.10-8.04 (m, 1H), 7.64 (s, 1H), 7.50-7.45 (m, 1H), 7.44-7.40 (m, 1H), 7.34-7.24 (m, 2H), 7.09-7.04 (m, 2H), 7.00-6.95 (m, 2H), 2.88-2.78 (m, 4H), 2,33 (s, 3H), 1.78-1.68 (m, 4H), 1.46-1.36 (m, 2H).
HRMS Calcd for [C₂₇H₂₅Cl₂NO₄S+H]⁺: 586.108. Found: 586,111.

### Step B: 4-{1-(2-4-dichlorophenyl)-4-methyl-3-[(piperidin-1-ylamino)carbonyl-]1H-pyrazol-5-yl}phenyl pyridine-3-sulfonate hydrochloride

4-{1-(2,4-dichlorophenyl)-4-methyl-3-[(piperidin-1-ylamino)carbonyl]-1*H*-pyrazol-5-yl)phenyl pyridine-3-sulfonate (150 mg, 0.26 mmol) was dissolved in 0.1 M HCl in acetic acid (10 ml) and freeze dried to give the salt as a white powder (159 mg, 99.8%).

### Reference Example 14

### tert-butyl [2-(4-{1-(2,4-dichlorophenyl)-4-methyl-3-[(piperidin-1-ylamino)carbonyl]-1H-pyrazol-5-yl}phenoxy)ethyl]ethylcarbamate

### Step A: tert-butyl ethyl(2-hydroxyetyl)carbamate

Di-tert-butyl dicarbonate (3.19 g, 14.6 mmol) in THF (10 ml) was added to 2-(ethylamino)ethanol (1.00 g, 11.2 mmol) and reacted at room temperature for 3 hours, The solvent was evaporated at reduced pressure to give the crude product (2.28 g).
¹H NMR (499.961 MHz) δ 3.71-3.65 (br, 2H), 3.55-3.35 (br, 1H), 3.35-3.30 (br, 2H), 3.30-3.20 (br, 2H), 1.43 (s, 9H), 1.07 (t, 3H).

### Step B: tert-butyl[2-(4-{1-(2,4-dichlorophenyl)-4-methyl-3-[(piperidin-1-ylamino)carbonyl]-1H-pyrazol-5-yl}phenoxy)ethyl]ethylcarbamate

1-(2,4-dichlorophenyl)-5-(4-hydroxyphenyl)-4-metyl-*N*-piperidin-1-yl-1*H*-pyrazole-3-carboxamide, prepared as Ex 1., Step F (151.6 mg, 0.34 mmol) and *tert*-butyl ethyl(2-hydraxyethyl)carbamate (408.6 mg, 1.73 mmol) were mixed in toluene (1ml) and reacted repeatedly in a single node microwave oven at 180°C. The total reaction time was 2 hours. Cyanomethylenetri-N-butylphosphorane was added before each heating (total amount 925 mg, 3.83 mmol). The solvent was then evaporated and the product purified by preparatory HPLC (kromasil C8 column, ammonium acetate (aq, 0.1 M):acetonitrile, product came at 100% acetonitrile) and flash chromatography (SiO₂, toluene: ethylacetate, product came at 30% ethyl acetate) to give an almost white powder (125 mg, 60%).
¹HNMR (399.964 MHz) δ 7.62 (s, 1H). 7.37 (s, 1H), 7.23 (s, 2H), 7.01,6.95 (m, 2H), 6.81-6.75 (m, 2H), 4.05-3.95 (br, 2H), 3.55-3.45 (br, 2H), 3.35-3.25 (br, 2H), 2.86-2.78 (br, 4H), 2.32 (s, 3H), 1.75-1.65 (m, 4H), 1.41 (s, 9H), 1.45-1.35 (m, 2H), 1.08 (t, 3H).
MS *m*/*z* 616, 618, 620 (M+H)⁺.

### Reference Example 15

### 1-(2,4-dichlorophenyl)-5-{4-[2(ethylamino)ethoxy]phenyl}-4-methyl-N-piperidin-1-yl-1H-pyrazole-3-carboxamide dihydrochloride

Thionyl chloride (1 ml, 13.71 mmol) in methanol (10 ml) was added to a suspension of *tert*-butyl [2-(4-{1-(2,4-dichlorophenyl)-4-methyl-3-[(piperidin-1-ylamino)carbonyl]-1*H-*pyrazol-5-yl}phenoxy)ethyl]ethylcarbamate from Ex 14, Step B (137 mg, 0.22 mmol) in methanol (2 ml). After 2.5 hours the solvent was evaporated and the product freeze dried, The compound was purified by preparatory HPLC (kromasil C8 column, ammonium acetate (aq, 0.1 M):acetonitrile, product came at 72% acetonitrile), freeze dried and finally dissolved in 0,1 M HCl in acetic acid (15 ml) and freeze dried again to give an almost white powder (53 mg, 40%).
¹H NMR (399.964 MHz) δ 7.54-7.47 (m, 2H), 7.44-7.39 (m, 1H), 7.19-7.14 (m, 2H), 7.01-6.96 (m, 2H), 4.24 (t, 2H), 3.41 (t, 2H), 3.18-3.06 (m, 6H), 2.27 (s, 3H), 1.87-1.78 (m, 4H), 1.57-1.48 (m, 2H), 1.32 (t, 3H).

HRMS Calcd for [C₂₆H₃₁Cl₂N₅O₂+H]⁺: 516.193. Found: 516.195.

### Example 16

### 4-{1-(2,4-dichlorophenyl)-4-methyl-3-[(piperidin-1-ylamino)carbonyl]-1H-pyrazol-5-yl}phenyl 3-methylbutane-1-sulfonate

3-Methylbutane-1-sulfonyl chloride (84 mg, 0.49 mmol) in DCM (2 ml) was added to a mixture of 1-(2,4-dichlorophenyl)-5-(4-hydroxyphenyl)-4-methyl-*N-*piperidin-1-yl-1*H-*pyrazole-3-carboxamide, prepared as in Example 1, Step F (100 mg, 0.23 mmol) and TEA (0.5 ml, 3.59 mmol) in DCM (2 ml) at -78°C, under N₂(g). The reaction was continued at - 78°C for 1 hour. Water was added and the phases were separated. The organic phase was washed with water and dried over MgSO₄. The product was further purified by preparatory HPLC (kromasil C8 column, ammonium acetate (aq, 0.1 M):acetonitrile, product came at 100% acetonitrile) to give an almost white powder (94 mg, 72%).
¹H NMR (399.964 MHz) δ 7.85-7.45 (br, 1H), 7.38 (s, 1H), 7.29-7.25 (m, 2H), 7.23-7.18 (m, 2H), 7.16-7.11 (m, 2H), 3.25-3.18 (m, 2H), 2.88-2.80 (m, 4H), 2.33 (s, 3H), 1.86-1.77 (m, 2H), 1.76-1.65 (m, 5H), 1.45-1.34 (m, 2H), 0.92 (d, 6H).
HRMS Calcd for [C₂₇H₃₂Cl₂N₄O₄S+H]⁺: 579.160. Found: 579.159.

### Example 17

### 4-{1-(2,4-dichlorophenyl)-4-methyl-3-[(piperidin-1-ylamino)carbonyl]-1H-pyrazol-5-yl}phenyl 3,3-dimethylbutane-1-sulfonate

3,3-Dimethylbutane-1-sulfonyl chloride (59 mg, 0.32 mmol) in DCM (2 ml) was added to a mixture of 1-(2,4-dichlorophenyl)-5-(4-hydroxyphenyl)-4-methyl-*N*-piperidin-1-yl-1*H-*pyrazole-3-carboxamide, prepared as in Example 1, Step F (103 mg, 0.23 mmol) and TEA (0.5 ml, 3.59 mmol) in DCM (2 ml) at -78°C, under N₂(g). The reaction was continued at - 78°C for 1 hour. Water was added and the phases were separated. The organic phase was washed with water and dried over MgSO₄. The product was further purified by preparatory HPLC (kromasil C8 column, ammonium acetate (aq, 0.1 M):acetonitrile, product came at 100% acetonitrile) to give an almost white powder (94 mg, 68%).
¹H NMR (399.964 MHz) δ 8.20-7.40 (br, 1H), 7.36 (s, 1H), 7.28-7.22 (m, 2H), 7.21-7.16 (m, 2H), 7.15-7.09 (m, 2H), 3.20-3.13 (m, 2H), 2.87-2.80 (m, 4H), 2.31 (s, 3H), 1.83-1.76 (m, 2H), 1.73-1.65 (m, 4H), 1.43-1.32 (m, 2H), 0.89 (s, 9H).
HRMS Calcd for [C₂₈H₃₄Cl₂N₄O₄S+H]⁺: 593.176, Found: 593.176.

### Example 18

### 4-[1-(2,4-dichlorophenyl)-4-methyl-3-({5-(trifluoromethyl)pyridin-2-yl]amino}carbonyl)-1H-pyrazol-5-yl]phenyl 3,3,3-trifluoropropane-1-sulfonate

### Step A: 5-[4-(benzyloxy)phenyl]-1-(2,4-dichlorophenyl-4-methyl-N-[5-(trifluoromethyl)pyridin-2-yl]-1H-pyrazole-3-carboxamide

Oxalyl chloride (1 ml) in DCM (4 ml) was added to a suspension of 5-[4-(benzyloxy)phenyl]-1-(2,4-dichlorophenyl)-4-methyl-1*H*-pyrazole-3-carboxylic acid, prepared as in Example 1, Step D (202 mg, 0.45 mmol) in DCM (4 ml). 1 drop of DMF was added and the reaction continued at room temperature for 1 hour. DCM and excess oxalyl chloride were removed at reduced pressure and the acid chloride suspended in DCM (4 ml) and added to 5-(trifluoromethyl)pyridine-2-amine (87.6 mg, 0.54 mmol) in DCM/K₂CO₃ (10%, aq) (1/1, 8 ml). The reaction was continued at room temperature for 24 hours. Then 50 mg DMAP was added and the reaction continued at room temperatue for 1 hour. The phases were separated and the organic phase washed with water and dried over MgSO₄. The product was purified further by flash chromatography (SiO₂, toluene) (108 mg, 41%).
¹H NMR (399.964 MHz) δ 9.66 (s, 1H), 8.58-8.48 (m, 2H), 7.95-7.90 (m, 1H), 7.44-7.14 (m, 8H), 7.10-7.04 (m, 2H), 6.94-6.90 (m, 2H), 5.02 (s, 2H), 2.42 (s, 3H).
MS *m*/*z* 597, 599, 601 (M+H)⁺.

### Step B: 1-(2,4-dichlorophenyl)-5-(4-hydroxyphenyl)-4-methyl-N-[5-(trifluoromethyl)pyridine-2-yl]-1H-pyrazole-3-carboxamide

5-[4-(benzyloxy)phenyl]-1-(2,4-dichlorophenyl)-4-methyl-*N*-[5-(trifluoromethyl)pyridin-2-yl]-1*H-*pyrazole-3-carboxamide (108 mg, 0.18 mmol) was suspended in 4.1 M HBr in acetic acid (10 ml) and reacted at room temperature for 6 hours. Ethanol was added (95%, 75 ml) and the solvent evaporated at reduced pressure. Methanol was added and the product was neutralised with NaHCO₃ (1M, aq). The solvent was evaporated and the mixture dissolved in DCM and water. The phases were separated and the organic phase washed with water and dried over MgSO₄ (87 mg, 95%).
¹H NMR (399,964 MHz) δ 9.64 (s, 1H), 8.58-8.48 (m, 2H), 7.97-7.91 (m, 1H), 7.45-7.41 (m, 1H), 7.32-7.23 (m, 2H), 7.03-6.98 (m, 2H), 6.80-6.75 (m, 2H), 5.50-5.30 (br, 1H), 2.40 (s, 3H).
MS *m*/*z* 507, 509, 511 (M+H)⁺.

### Step C: 4-[1-(2,4-dichlorophenyl)-4-metyl-3-({[5-(trifluoromethyl)pyridin-2-yl]amino}carbonyl)-1H-pyrazol-5-yl]phenyl 3,3,3-trifluoropropane-1-sulfonate

3,3,3-trifluoropropane-1-sulfonyl chloride (105 mg, 0.53 mmol) was added to a mixture of 1-(2,4-dichlorophenyl)-5-(4-hydroxyphenyl)-4-methyl-*N-*[5-(trifluoromethyl)pyridine-2-yl]-1*H*-pyrazole-3-carboxamide (87 mg, 0.17 mmol) and TEA (0.5 ml, 3,59 mmol) in DCM (3 ml) at -78°C, under N₂(g). The reaction was continued at -78°C for 2 hours and then at room temperature over night. Water was added and the phases were separated. The organic phase was washed with water and dried over MgSO₄. The product was further purified by preparatory HPLC (kromasil C8 column, ammonium acetate (aq, 0.1 M):acetonitrile, product came at 100% acetonitrile) to give an almost white powder (86 mg, 75%).
¹H NMR (399.964 MHz) δ 9.70 (s, 1H), 8.57-8.48 (m, 2H), 7.99-7.92 (m, 1H), 7.43 (s, 1H), 7.32 (s, 2H), 7,28-7.19 (m, 4H), 3.53-3.44 (m, 2H), 2.85-2,71 (m, 2H), 2.42 (s, 3H). HRMS Calcd for [C₂₆H₁₈Cl₂F₆N₄O₄S+H]⁺: 667.041. Found: 667.031.

### Reference Example 19

### 1-(2,4-dichlorohenyl)-5-{4-[2-(1,3-dioxolan-2-yl)ethoxyl]phenyl}-4-methyl-N-piperidin-1-yl-1H-pyrazole-3-carboxamide

2-(2-bromoethyl)-1,3-dioxolane (60 mg, 0.33 mmol), 1-(2,4-dichlorophenyl)-5-(4-hydroxyphenyl)-4-methyl-*N*-piperidin-1-yl-1*H*-pyrazole-3-carboxamide prepared as in Ex.1, Step F (100 mg, 0.22 mmol) and potassium carbonate (150 mg, 1,09 mmol) were refluxed in acetonitrile (25 ml) for 15 hours. The solvent was evaporated, water and DCM were added, the phases separated and the organic phase washed with water and dried (MgSO₄). The product was further purified by preparatory HPLC (kromasil C8 column, ammonium acetate (aq, 0.1 M):acetonitrile, product came at 100% acetonitrile) to give an almost white powder (60 mg, 49%).
¹H NMR (399.964 MHz) δ 7.80-7.50 (br, 1H), 7.36 (s, 1H), 7.21 (s, 2H), 7.00-6.94 (m, 2H), 6.80-6.74 (m, 2H), 5.02 (t, 1H), 4.04 (t, 2H), 3.96-3.78 (m, 4H), 2.56-2.78 (m, 4H), 2.30 (s, 3H), 2.09 (q, 2H), 1.74-1.b5 (m, 4H), 1.42-1.32 (m, 2H).

HRMS Calcd for [C₂₇H₃₀Cl₂N₄O₄+H]⁺: 545.172. Found: 545.172.

### Example 20

### Propane-1-sulfonic acid 4-[2-(2,4-dichloro-3-fluorophenyl)-4-methyl-5-(piperidin-1-ylcarbamoyl)-2H-pyrazol-3-yl]phenyl ester

### Step A: 1-(2,4-Dichloro-3-fluorophenyl)-5-(4-methoxyphenyl)-4-methyl-1H-pyrazole-3-carboxylic acid

A solution of sodium ethoxide was generated from sodium metal (0.18 g, 7.89 mmol) in 20 ml abs. ethanol. To this solution was added 2-acetyl-4-(4-methoxy-phenyl)-3-methyl-4-oxo-butyric acid ethyl ester (1.73 g, 5.92 mmol) and the reaction mixture stirred at room temperature for 30 min. A previously prepared solution of 2,4-dichloro-3-fluorodiazonium chloride (prepared from 2,4-dichloro-3-fluoroaniline (1.30 g, 7.22 mmol) in 3 ml 24% HCl and sodium nitrite (0.51 g, 7.39 mmol) in 3.5 ml water at 0 °C) was added in 5 portions keeping the temperature below 5 °C. After stirring at room temperature for 2.5 hours water was added, the product extracted with EtOAc (3 x). The combined organic extracts was dried (Na₂SO₄), filtered and evaporated. The residue was dissolved in ethanol (40 ml) and sodium hydroxide (1.00 g, 25.0 mmol) in 5 ml of water was added. After 2 hours boiling under reflux the reaction mixture was cooled, acidified with HCl and the product extracted with EtOAc (3 x). After washing, drying (Na₂SO₄), filtration and concentration, the residue was purified by flash chromtography (hexane : EtOAc 70:30 - 50:50) affording 1.37 g (31%) of the title compound as a light brown solid.

### Step B: 1-(2,4-Dichloro-3-fluoro-phenyl)-5-(4-methoxy-phenyl)-4-methyl-1H-pyrazole-3-carboxylic acid piperidin-1-ylamide

1-(2,4-Dichloro-3-fluoro-phenyl)-5-(4-methoxy-phenyl)-4-methyl-1*H*-pyrazole-3-carboxylic acid (1.37 g, 3.43 mmol) was suspended in dichloromethane (20 ml) and a few drops of DMF added followed by the addition of oxalylchloride (0.87 g, 6.86 mmol). The reaction mixture was refluxed for two hours. After cooling to room temperature the solvent was removed and the crude acid chloride redissolved in dichloromethane (20 ml), cooled to 0°C, Et₃N (0.96 ml, 6.94 mmol) was added followed by 1-aminopiperidine (0.37 ml, 3.62 mmol). The cooling bath was removed and the reaction mixture stirred at room temperature overnight. Water was added and the product extracted with dichloromethane (3x), the combined extracts dried (Na₂SO₄), filtered and evaporated. Flash chromatography (hexane ; EtOAc 50 : 50) afforded 0.79 g (48%) of the title compound as a light brown solid.

### Step C: 1-(2,4-Dichloro-3-fluoro-phenyl)-5-(4-hydroxyl-phenyl)-4-methyl-1H-pyrazole-3-carboxylic acid piperidin-1-ylamide

1-(2,4-Dichloro-3-fluoro-phenyl)-5-(4-methoxy-phenyl)-4-methyl-1*H*-pyrazole-3-carboxylic acid piperidin-1-ylamide (0.79 g, 1.66 mmol) was dissolved in 40 ml of dichloromethane at 0 °C. Boron tribromide (0.32 ml, 3,31 mmol) was added, the cooling bath was removed and stirring continued for 2 hrs at room temperature before pouring it onto ice-water and extracting with DCM (x3). The combined extracts were dried (Na₂SO₄), filtered and concentrated. Flash chromatography (EtOAc) afforded 0.36 g (47%) of the title compound as a colorless solid.

### Step: Propane-1-sulfonic acid 4-[2-(2,4-dichloro-3-fluoro-phenyl)-4-methyl-5-(piperidin-1-ylcarbamoyl)-2H-pyrazol-3-yl-phenyl ester

To a solution of 1-(2,4-dichloro-3-fluoro-phenyl)-5-(4-hydroxy-phenyl)-4-methyl-1*H-*pyrazole-3-carboxylic acid piperidin-1-ylamide (0.36 g, 0.78 mmol) in dichloromethane (10 ml) was added triethylamine (0.22 ml, 1.56 mmol) and the reaction mixture cooled to 0 °C. 1-Propanesulfonylchloride (0.22 g, 1.56 mmol) was added, the cooling bath removed and the reaction mixture stirred at rt for 2 hrs. Water was added, the product extracted with DCM (x2), the combined organic extracts washed with water, dried (Na₂SO₄), filtered and concentrated. Flash chromatography (hexane : EtOAc gradient) afforded 100 mg (23%) of the title compound as a colorless solid. HPLC 80% purity. Preparative HPLC afforded 40 mg of the title compound.
¹H NMR (CDCl₃); δ 7.50-7.20 (7 H, m), 3.57-3.23 (6H, m), 2.37 (3H, s),2.10-1.80 (6H, m), 1.70-1.50 (2H, m), 1.10 (3H, t)
MS: 591 (M+Na)
HPLC: 97.1 %

### Reference Example 21

### 5-Chlorothiophene-2-sulfonic acid 4-[2-(2,4-dichlorophenyl)-4-methyl-5-(piperidin-1-ylcarbamoyl)-2H-pyrazol-3-yl]phenyl ester

1-(2,4-Dichloro-phenyl)-5-(4-hydroxy-phenyl)-4-methyl-1H-pyrazole-3-carboxylic acid piperidin-1-ylamide, prepared as in Ex 1, Step F (100 mg, 0.22 mmol) was dissolved in dry dichloromethane (3 ml) under nitrogen atmosphere and triethyl amine (0.09 ml) was added. 5-Chlorothiophene-2-sulfonyl chloride (0.54 mg, 0.24 mmol), dissolved in dry dichloromethane (2 ml), was then added. The reaction mixture was stirred over the weekend at room temperature. The reaction mixture was diluted with dichloromethane to 20 ml and was then washed with water (2x5 ml) and brine (5 ml). The organic layer was concentrated under reduced pressure to give an oil. The crude material was purified by reverse phase HPLC, Kromasil C8, 5-100% MeCN in water with 0.1M ammonium acetate. The product fraction was concentrated under reduced pressure and the residue was dissolved in dichloromethane and washed with water and brine. The organic layer was dried (MgSO₄), filtered and concentrated under reduced pressure to give the title compound as a yellow solid.
¹H-NMR (CDCl₃): δ 1.42-1.50 (m, 2H), 1.73-1.81 (m, 4H), 2.39 (s, 3H), 2.84-2.92 (m, 4H), 6.95 (d, 1H), 7.04-7.14 (m, 4H), 7.30-7.36 (m, 3H), 7.44 (d, 1H), 7.63 (s, 1H).
MS: 625 (M+1). HPLC: >99% purity.

## Claims

1. A compound of formula IA 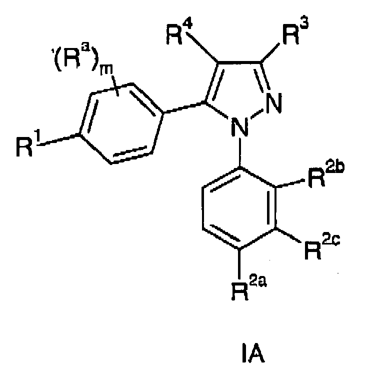 or a pharmaceutically acceptable salt thereof, in which
in which R¹ is a group R⁵S(O)₂O in which R⁵ represents a C₁₋₆alkyl group optionally substituted by one or more fluoro;
R^{a} represents halo and m is 0, 1 or 2;
R^{2a} represents chloro;
R^{2b} represents chloro;
R^{2c} represents H or fluoro;
R³ represents a group CONHNR⁷R⁸ in which NR⁷R⁸ represents piperidino or morpholino or R³ represents a group CONHR⁸ in which R⁸ represents a C₅₋₇cycloalkyl group optionally substituted by a C₁₋₆alkoxycarbonyl group or R³ represents 5-(trifluoromethyl)pyridin-2-ylaminocarbonyl; and
R⁴ represents H, a C₁₋₃alkyl group or halo.

2. A compound as claimed in claim 1, as represented by formula IB 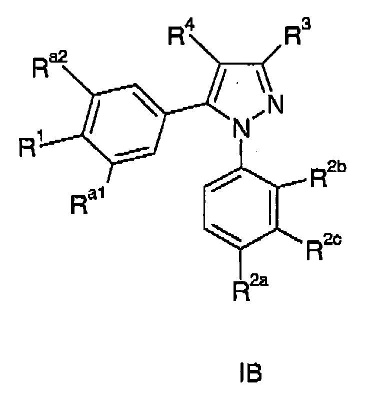 or a pharmaceutically acceptable salt thereof,
in which R¹ is a group R⁵S(O)₂O in which R⁵ represents a C₁₋₆alkyl group optionally substituted by one or more fluoro;
R^{a1} represents halo or H;
R^{a2} represents halo or H;
R^{2a} represents chloro;
R^{2b} represents chloro;
R^{2c} represents halo or H;
R³ represents a group CONHNR⁷R⁸ in which NR⁷R⁸ represents piperidino; and
R⁴ represents a C₁₋₃alkyl group,

3. A compound as claimed in claim 1 as represented by formula IC 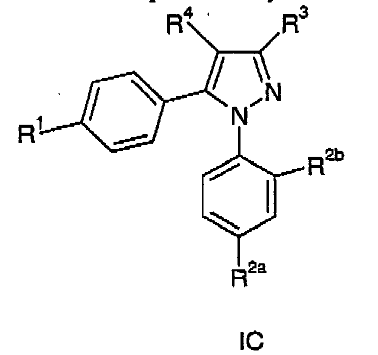 or a pharmaceutically acceptable salt thereof,
in which R¹ is a group R⁵S(O)₂O in which R⁵ represents a C₁₋₆alkyl group optionally substituted by one or more fluoro;
R^{2a} represents chloro;
R^{2b} represents chloro;
R³ represents a group CONHNR⁷R⁸ in which NR⁷R⁸ represents piperidino or morpholino; and
R⁴ represents a C₁₋₃alkyl group or halo.

4. A compound as claimed in claim 1 selected from one or more of the following: butane-1-sulfonic acid 4-[2-(2,4-dichlorophenyl)-4-methyl-5-(piperidin-1-ylcarbamoyl)-2*H*-pyrazol-3-yl]phenyl ester;
propane-1-sulfonic acid 4-[2-(2,4-dichlorophenyl)-4-methyl-5-(piperidin-1-ylcarbamoyl)2*H*-pyrazol-3-yl]phenyl ester;
propane-1-sulfonic acid 4-[2-(2,4-dichlorophenyl)-4-methyl-5-(morpholin-4-ylcarbamoyl)-2*H*-pyrazol-3-yl-phenyl ester;
3,3,3-trifluoropropane-1-sulfonic acid 4-[2-(2,4-dichloxophenyl)-4-methyl-5-(piperidin-1-ylcarbamoyl)-2*H*-pyrazol-3-yl]phenyl ester;
4,4,4-trifluorobutane-1-sulfonic acid 4-[2-(2,4-dichlorophenyl)-4-methyl-5-(piperidin-1-ylcarbamoyl)-2*H*-pyrazol-3-yl]phenyl ester;
propane-1-sulfonic acid-4-[2-(2,4-dichlorophenyl)-4-methyl-5-(piperidin-1-ylcarbamoyl)-2*H*-pyrazol-3-yl]-2,6-difluorophenyl ester;
progane-1-sulfonic acid 4-[2-(2,4-dichlorophenyl)-5-(piperidin-1-ylcarbamoyl)-2*H-*pyrazol-3-yl]phenyl ester;
propane-1-sulfonic acid 4-[4-bromo-2-(2,4-dichlorophenyl)-5-(piperidin-1-ylcarbamoyl)-2*H*-pyrazol-3-yl]phenyl ester;
methyl 1-{[(1-(2,4-dichlorophenyl)-4-methyl-5-{4[(propylsulfonyl)oxy]phenyl}-1*H-*pyrazol-3-yl)carbonyl] amino}cyclopentanecarboxylate;
4-{1-(2,4-dichlorophenyl)-4-methyl-3-[(piperidin-1-ylamino)carbonyl]-1*H*-pyrazol-5-yl}phenyl3-methylbutane-1-sulfonate;
4-{1-(2,4-dichlorophenyl)-4-methyl-3-[(piperidin-1-ylamino)carbonyl]-1*H*-pyrazol-5-yl}phenyl 3,3-dimethylbutane-1-sulfonate;
4-[1-(2,4-dichlorophenyl)4-methyl-3-({[5-(trifluoromethyl)pyridin-2-yl]amino}carbonyl)-1*H*-pyrazol-5-yl]phenyl3,3,3-trifluoropropane-1-sulfonate; and
propane-1-sulfonic acid 4-[2-(2,4-dichloro-3-fluorophenyl)-4-methyl-5-(piperidin-1-ylcarbamoyl)-2*H*-pyrazol-3-yl]phenyl ester; or a pharmaceutically acceptable salt thereof.

5. A compound of formula I as claimed in any one of claims 1 to 4 for use as a medicament.

6. A pharmaceutical formulation comprising a compound of formula I as claimed in any one of claims 1 to 4 and a pharmaceutically acceptable adjuvant, diluent or carrier.

7. Use of a compound of formula I as claimed in as claimed in any one of claims 1 to 4 in the preparation of a medicament for the treatment or prophylaxis of obesity, psychiatric disorders such as psychotic disorders, schizophrenia and bipolar disorders, anxiety, anxio-depressive disorders, depression, cognitive disorders, memory disorders, obsessive-compulsive disorders, anorexia, bulimia, attention disorders, epilepsy and related conditions, and neurological disorders, Parkinson's Disease, Huntington's Chorea and Alzheimer's Disease, immune, cardiovascular, reproductive and endocrine disorders, septic shock, diseases related to the respiratory and gastrointestinal systems, and extended abuse, addiction and/or relapse indications.

8. A compound as defined in as claimed in any one of claims 1 to 4 for use in the treatment of obesity.

9. A process for the preparation of a compound of formula IA according to claim 1 comprising reacting a compound of formula IIIA 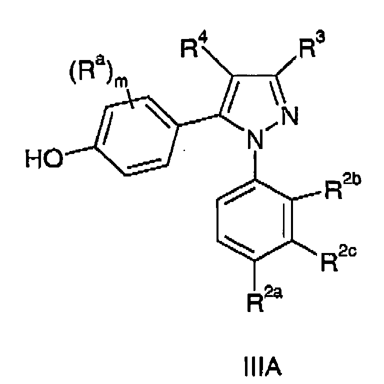 in which R^{a}, R^{2a}, R^{2b}, R^{2c}, R³, R⁴ and m are as previously defined with a sulphonating agent of formula R⁵SO₂L in which R⁵ is as previously defined and L represents a leaving group in an inert solvent in the presence of a base at a temperature in the range of -25°C, to 150°C.

## Patentansprüche

1. Verbindung der Formel IA 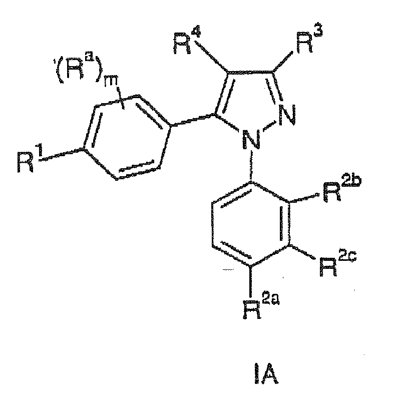 oder ein pharmazeutisch annehmbares Salz davon, worin
R¹ für eine Gruppe R⁵S (O)₂O, worin R⁵ eine gegebenenfalls durch ein oder mehrere Fluoratome substituierte C₁₋₆-Alkylgruppe bedeutet, steht;
R^{a} für Halogen steht und m für 0, 1 oder 2 steht;
R^{2a} für Chlor steht;
R^{2b} für Chlor steht;
R^{2c} für H oder Fluor steht;
R³ für eine Gruppe CONHNR⁷R⁸, worin NR⁷R⁸ Piperidino oder Morpholino bedeutet, eine Gruppe CONHR⁸, worin R⁸ eine gegebenenfalls durch eine C₁₋₆-Alkoxycarbonylgruppe substituierte C₅₋₇-Cycloalkylgruppe bedeutet, oder 5-(Trifluormethyl)pyridin-2-yl-aminocarbonyl steht und
R⁴ für H, eine C₁₋₃-Alkylgruppe oder Halogen steht.

2. Verbindung nach Anspruch 1 gemäß Formel IB 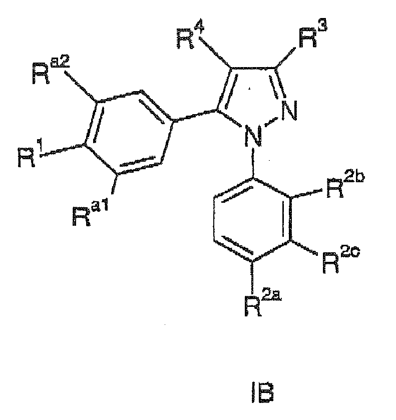 oder ein pharmazeutisch annehmbares Salz davon, worin
R¹ für eine Gruppe R⁵S(O)₂O, worin R⁵ eine gegebenenfalls durch ein oder mehrere Fluoratome substituierte C₁₋₆-Alkylgruppe bedeutet, steht;
R^{a1} für Halogen oder H steht;
R^{a2} für Halogen oder H steht;
R^{2a} für Chlor steht;
R^{2b} für Chlor steht;
R^{2c} für Halogen oder H steht;
R³ für eine Gruppe CONHNR⁷R⁸, worin NR⁷R⁸ Piperidino bedeutet, steht und
R⁴ für eine C₁₋₃-Alkylgruppe steht.

3. Verbindung nach Anspruch 1 gemäß Formel IC 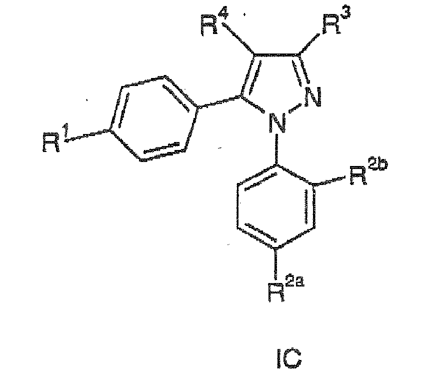 oder ein pharmazeutisch annehmbares Salz davon, worin
R¹ für eine Gruppe R⁵S(O)₂O, worin R⁵ eine gegebenenfalls durch ein oder mehrere Fluoratome substituierte C₁₋₆-Alkylgruppe bedeutet, steht;
R^{2a} für Chlor steht;
R^{2b} für Chlor steht;
R³ für eine Gruppe CONHNR⁷R⁸, worin NR⁷R⁸ Piperidino oder Morpholino bedeutet, steht und
R⁴ für eine C₁₋₃-Alkylgruppe oder Halogen steht.

4. Verbindung nach Anspruch 1, ausgewählt unter einer oder mehreren der folgenden Verbindungen:
Butan-1-sulfonsäure-4-[2-(2,4-dichlorphenyl)-4-methyl-5-(piperidin-1-ylcarbamoyl)-2*H*-pyrazol-3-yl]phenylester;
Propan-1-sulfonsäure-4-[2-(2,4-dichlorphenyl)-4-methyl-5-(piperidin-1-ylcarbamoyl)-2*H*-pyrazol-3-yl]phenylester;
Propan-1-sulfonsäure-4-[2-(2,4-dichlorphenyl)-4-methyl-5-(morpholin-4-ylcarbamoyl)-2*H*-pyrazol-3-yl]phenylester;
3,3,3-Trifluorpropan-1-sulfonsäure-4-[2-(2,4-dichlorphenyl)-4-methyl-5-(piperidin-1-yl-carbamoyl)-2*H*-pyrazol-3-yl]phenylester;
4,4,4-Trifluorbutan-1-sulfonsäure-4-[2-(2,4-dichlorphenyl)-4-methyl-5-(piperidin-1-yl-carbamoyl)-2*H*-pyrazol-3-yl]phenylester;
Propan-1-sulfonsäure-4-[2-(2,4-dichlorphenyl)-4-methyl-5-(piperidin-1-ylcarbamoyl)-2*H*-pyrazol-3-yl]-2,6-difluorphenylester;
Propan-1-sulfonsäure-4-[2-(2,4-dichlorphenyl)-5-(piperidin-1-ylcarbamoyl)-2*H*-pyrazol-3-yl]phenylester;
Propan-1-sulfonsäure-4-[4-brom-2-(2,4-dichlorphenyl)-5-(piperidin-1-ylcarbamoyl)-2*H*-pyrazol-3-yl]phenylester;
1-{[(1-(2,4-Dichlorphenyl)-4-methyl-5-{4-[(propyl-sulfonyl)oxy]phenyl}-1*H*-pyrazol-3-yl)carbonyl]-amino}cyclopentancarbonsäuremethylester;
4-{1-(2,4-Dichlorphenyl)-4-methyl-3-[(piperidin-1-ylamino)carbonyl]-1*H*-pyrazol-5-yl}phenyl-3-methylbutan-1-sulfonat;
4-{1-(2,4-Dichlorphenyl)-4-methyl-3-[(piperidin-1-ylamino)carbonyl]-1*H*-pyrazol-5-yl}phenyl-3,3-dimethylbutan-1-sulfonat;
4-{1-(2,4-Dichlorphenyl)-4-methyl-3-({[5-(trifluormethyl)pyridin-2-yl]amino}carbonyl)-1*H-*pyrazol-5-yl}phenyl-3,3,3-trifluorpropan-1-sulfonat und
Propan-1-sulfonsäure-4-[2-(2,4-dichlor-3-fluorphenyl)-4-methyl-5-(piperidin-1-ylcarbamoyl)-2*H-*pyrazol-3-yl]phenylester;
oder ein pharmazeutisch annehmbares Salz davon.

5. Verbindung der Formel I nach einem der Ansprüche 1 bis 4 zur Verwendung als Arzneimittel.

6. Pharmazeutische Formulierung, enthaltend eine Verbindung der Formel I nach einem der Ansprüche 1 bis 4 und einen pharmazeutisch annehmbaren Hilfsstoff, ein pharmazeutisch annehmbares Verdünnungsmittel oder einen pharmazeutisch annehmbaren Träger.

7. Verwendung einer Verbindung der Formel I nach einem der Ansprüche 1 bis 4 bei der Herstellung eines Arzneimittels zur Behandlung oder Prophylaxe von Obesitas, psychiatrischen Störungen wie psychotischen Störungen, Schizophrenie und manisch-depressiven Psychosen, Angst, anxiodepressiven Störungen, Depression, kognitiven Störungen, Gedächtnisstörungen, Zwangsneurosen, Anorexie, Bulimie, Aufmerksamkeitsstörungen, Epilepsie und verwandten Leiden, und neurologischen Störungen, Parkinson-Krankheit, Chorea Huntington und Alzheimer-Krankheit, Erkrankungen des Immunsystems, des Herzkreislaufsystems, der Fortpflanzungsorgane und des Endokrinsystems, septischem Schock, mit den Atemwegen und dem Magen-Darm-Trakt in Zusammenhang stehenden Erkrankungen und Indikationen von anhaltendem Substanzmißbrauch, Sucht und/oder Rückfall.

8. Verbindung nach einem der Ansprüche 1 bis 4 zur Verwendung bei der Behandlung von Obesitas.

9. Verfahren zur Herstellung einer Verbindung der Formel IA nach Anspruch 1, bei dem man eine Verbindung der Formel IIIA 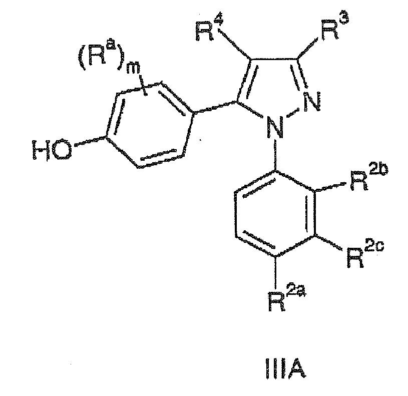 worin R^{a}, R^{2a}, R^{2b}, R^{2c}, R³, R⁴ und m die oben angegebene Bedeutung besitzen, in einem inerten Lösungsmittel in Gegenwart einer Base bei einer Temperatur im Bereich von -25°C bis 150°C mit einem Sulfonierungsmittel der Formel R⁵SO₂L, worin R⁵ die oben angegebene Bedeutung besitzt und L für eine Abgangsgruppe steht, umsetzt.

## Revendications

1. Composé de formule IA 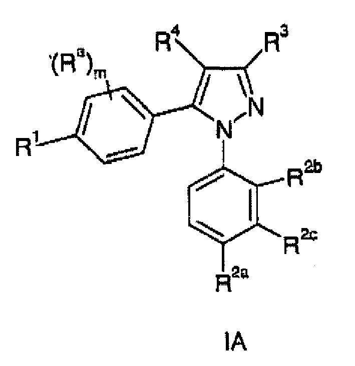 ou un sel pharmaceutiquement acceptable de celui-ci, dans laquelle
R¹ est un groupement R⁵S(O)₂O dans lequel R⁵ représente un groupement C₁₋₆alkyle éventuellement substitué par un ou plusieurs fluoro ;
R^{a} représente halogéno et m est 0, 1 ou 2 ;
R^{2a} représente chloro ;
R^{2b} représente chloro ;
R^{2c} représente H ou fluoro ;
R³ représente un groupement CONHNR⁷R⁸ dans lequel NR⁷R⁸ représente pipéridino ou morpholino ou R³ représente un groupement CONHR⁸ dans lequel R⁸ représente un groupement C₅₋₇cycloalkyle éventuellement substitué par un groupement C₁₋₆alcoxycarbonyle ou R³ représente 5-(trifluorométhyl)pyridin-2-ylaminocarbonyle ; et
R⁴ représente H, un groupement C₁₋₃alkyle ou halogéno.

2. Composé selon la revendication 1, tel que représenté par la formule IB 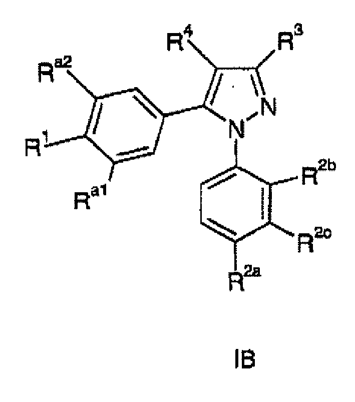 ou un sel pharmaceutiquement acceptable de celui-ci,
dans laquelle R¹ est un groupement R⁵S(O)₂O dans lequel R⁵ représente un groupement C₁₋₆alkyle éventuellement substitué par un ou plusieurs fluoro ;
R^{a1} représente halogéno ou H ;
R^{a2} représente halogéno ou H ;
R^{2a} représente chloro ;
R^{2b} représente chloro ;
R^{2c} représente halogéno ou H ;
R³ représente un groupement CONHNR⁷R⁸ dans lequel NR⁷R⁸représente pipéridino ; et
R⁴ représente un groupement C₁₋₃alkyle.

3. Composé selon la revendication 1, tel que représenté par la formule IC 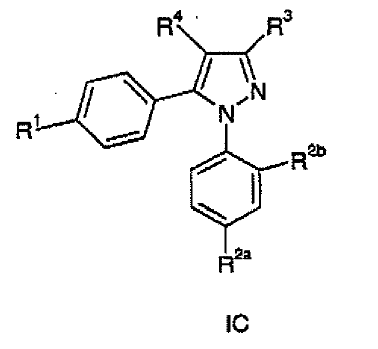 ou un sel pharmaceutiquement acceptable de celui-ci,
dans laquelle R¹ est un groupement R⁵S(O)₂O dans lequel R⁵ représente un groupement C₁₋₆alkyle éventuellement substitué par un ou plusieurs fluoro ;
R^{2a} représente chloro ;
R^{2b} représente chloro ;
R³ représente un groupement CONHNR⁷R⁸ dans lequel NR⁷R⁸ représente pipéridino ou morpholino ; et
R⁴ représente un groupement C₁₋₃alkyle ou halogéno.

4. Composé selon la revendication 1, **caractérisé en ce qu'**il est choisi parmi un ou plusieurs de ce qui suit :
l'ester 4-[2-(2,4-dichlorophényl)-4-méthyl-5-(pipéridin-1-ylcarbamoyl)-2*H*-pyrazol-3-yl]phénylique d'acide butane-1-sulfonique;
l'ester 4-[2-(2,4-dichlorophényl)-4-méthyl-5-(pipéridin-1-ylcarbamoyl]-2*H*-pyrazol-3-yl]phénylique d'acide propane-1-sulfonique ;
l'ester 4-[2-(2,4-dichlorophényl)-4-méthyl-5-(morpholin-4-ylcarbamoyl)-2*H*-pyrazol-3-yl]phénylique d'acide propane-1-sulfonique ;
l'ester 4-[2-(2,4-dichlorophényl)-4-méthyl-5-(pipéridin-1-ylcarbamoyl)-2*H*-pyrazol-3-yl]phénylique d'acide 3,3,3-trifluoropropane-1-sulfonique ;
l'ester 4-[2-(2,4-dichlorophényl)-4-méthyl-5-(pipéridin-1-ylcarbamoyl)-2*H*-pyrazol-3-yl]phénylique d'acide 4,4,4-trifluorobutane-1-sulfonique ;
l'ester 4-[2-(2,4-dichlorophényl)-4-méthyl-5-(pipéridin-1-ylcarbamoyl)-2*H*-pyrazol-3-yl]-2,6-difluorophénylique d'acide propane-1-sulfonique ;
l'ester 4-[2-(2,4-dichlorophényl)-5-(pipéridin-1-ylcarbamoyl)-2*H*-pyrazol-3-yl]phénylique d'acide propane-1-sulfonique ;
l'ester 4-[4-bromo-2-(2,4-dichlorophényl)-5-(pipéridin-1-ylcarbamoyl)-2*H*-pyrazol-3-yl]phénylique d'acide propane-1-sulfonique ;
le 1-{[(1-(2,4-dichlorophényl)-4-méthyl-5-{4[(propylsulfonyl)oxy]phényl}-1*H*-pyrazol-3-yl)carbonyl]amino}cyclopentanecarboxylate de méthyle ;
le 3-méthylbutane-1-sulfonate de 4-{1-(2,4-dichlorophényl)-4-méthyl-3-[(pipéridin-1-ylamino)carbonyl]-1*H*-pyrazol-5-yl}phényle ;
le 3,3-diméthylbutane-1-sulfonate de 4-{1-(2,4-dichlorophényl)-4-méthyl-3-[(pipéridin-1-ylamino)carbonyl]-1*H*-pyrazol-5-yl}phényle ;
le 3,3,3-trifluoropropane-1-sulfonate de 4[1-(2,4-dichlorophényl)-4-méthyl-3-({[5-(trifluorométhyl)pyridin-2-yl]amino}carbonyl)-1*H-*pyrazol-5-yl]phényle ; et
l'ester 4-[2-(2,4-dichloro-3-fluorophényl)-4-méthyl-5-(pipéridin-1-ylcarbamoyl)-2*H*-pyrazol-3-yl]phénylique d'acide propane-1-sulfonique ; ou un sel pharmaceutiquement acceptable de ceux-ci.

5. Composé de formule I selon l'une quelconque des revendications 1 à 4, destiné à être utilisé comme médicament.

6. Formulation pharmaceutique comprenant un composé de formule I selon l'une quelconque des revendications 1 à 4 et un adjuvant, un diluant ou un support pharmaceutiquement acceptable.

7. Utilisation d'un composé de formule I selon l'une quelconque des revendications 1 à 4, dans la préparation d'un médicament destiné au traitement ou à la prophylaxie de l'obésité, des troubles psychiatriques tels que les troubles psychotiques, la schizophrénie et les troubles bipolaires, de l'anxiété, des troubles anxio-dépressifs, de la dépression, des troubles cognitifs, des troubles mnésiques, des troubles obsessifs compulsifs, de l'anorexie, de la boulimie, des troubles de l'attention, de l'épilepsie et des affections associées, et des troubles neurologiques, de la maladie de Parkinson, de la chorée de Huntington et de la maladie d'Alzheimer, des troubles immunitaires, cardiovasculaires, de la reproduction et endocriniens, du choc septique, des maladies associées aux systèmes respiratoire et gastro-intestinal, et des indications d'abus prolongés, d'accoutumance et/ou de rechutes.

8. Composé tel que défini dans l'une quelconque des revendications 1 à 4, destiné à être utilisé dans le traitement de l'obésité.

9. Procédé de préparation d'un composé de formule IA selon la revendication 1, comprenant la réaction d'un composé de formule IIIA 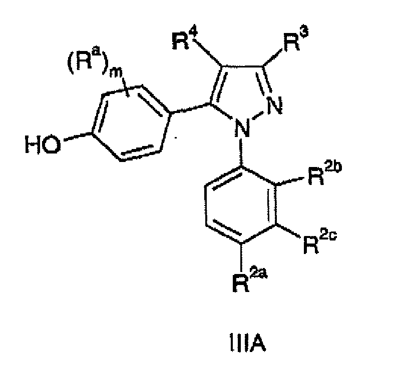 dans laquelle R^{a}, R^{2a}, R^{2b}, R^{2c}, R³, R⁴ et m sont tels que définis précédemment, avec un agent de sulfonation de formule R⁵SO₂L dans laquelle R⁵ est tel que défini précédemment et L représente un groupement partant, dans un solvant inerte en présence d'une base à une température dans la gamme de -25°C à 150°C.
